(19) Europäisches Patentamt / European Patent Office / Office européen des brevets

(11) **EP 4 413 504 B1**

(12) **EUROPEAN PATENT SPECIFICATION**

(45) Date of publication and mention
of the grant of the patent:
**27.08.2025 Bulletin 2025/35**

(21) Application number: **22797077.9**

(22) Date of filing: **29.09.2022**

(51) International Patent Classification (IPC):
**G06N 3/09** *(2023.01)*      **G16H 30/40** *(2018.01)*
**G16H 50/70** *(2018.01)*     G06N 5/01 *(2023.01)*
G06N 7/01 *(2023.01)*       G06N 20/10 *(2019.01)*
G06N 20/20 *(2019.01)*      G06N 3/0464 *(2023.01)*

(52) Cooperative Patent Classification (CPC):
**G16H 30/40; G06N 3/09; G16H 50/70;**
G06N 3/0464; G06N 5/01; G06N 7/01; G06N 20/10;
G06N 20/20

(86) International application number:
**PCT/EP2022/077226**

(87) International publication number:
**WO 2023/057321 (13.04.2023 Gazette 2023/15)**

(54) **UNCERTAINTY-BASED IN-PRODUCT LEARNING FOR X-RAY**

UNSICHERHEITSBASIERTES LERNEN IN EINEM PRODUKT FÜR RÖNTGENSTRAHLEN

APPRENTISSAGE PRODUIT BASÉ SUR L'INCERTITUDE POUR LES RAYONS X

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **05.10.2021 EP 21200958**

(43) Date of publication of application:
**14.08.2024 Bulletin 2024/33**

(73) Proprietor: **Koninklijke Philips N.V.
5656 AG Eindhoven (NL)**

(72) Inventors:
• **MAVROEIDIS, Dimitrios
5656 AG Eindhoven (NL)**
• **VDOVJAK, Richard
5656 AG Eindhoven (NL)**
• **SAALBACH, Axel
5656 AG Eindhoven (NL)**
• **LENGA, Matthias
5656 AG Eindhoven (NL)**

(74) Representative: **Philips Intellectual Property &
Standards
High Tech Campus 52
5656 AG Eindhoven (NL)**

(56) References cited:
• **FLORIN C GHESU ET AL: "Quantifying and
Leveraging Predictive Uncertainty for Medical
Image Assessment", ARXIV.ORG, CORNELL
UNIVERSITY LIBRARY, 201 OLIN LIBRARY
CORNELL UNIVERSITY ITHACA, NY 14853, 8
July 2020 (2020-07-08), XP081718282**
• **SUNGKWON AN ET AL: "OAAE: Adversarial
Autoencoders for Novelty Detection in Multi-
modal Normality Case via Orthogonalized Latent
Space", ARXIV.ORG, CORNELL UNIVERSITY
LIBRARY, 201 OLIN LIBRARY CORNELL
UNIVERSITY ITHACA, NY 14853, 7 January 2021
(2021-01-07), XP081853611**

EP 4 413 504 B1

**Description**

FIELD OF THE INVENTION

The invention broadly relates to machine learning. Specifically, invention relates to a system for providing training data for use in machine learning, to a method for providing training data, to an imaging arrangement, and to a computer program element and to a computer readable medium.

BACKGROUND OF THE INVENTION

Ghesu, F. C., Georgescu, B., Mansoor, A., Yoo, Y., Gibson, E., Vishwanath, R. S., ... & Comaniciu, D. (2021). Quantifying and leveraging predictive uncertainty for medical image assessment. Medical Image Analysis, 68, 101855 discloses a system that learns not only the probabilistic estimate for classification of medical input images, but also an explicit uncertainty measure which captures the confidence of the system in the predicted output.

An, S., Kim, J., Kang, M., Razaei, S., & Liu, X. (2021). OAAE: Adversarial Autoencoders for Novelty Detection in Multi-modal Normality Case via Orthogonalized Latent Space. arXiv preprint arXiv:2101.02358 discloses background art on novelty or anomaly detection using deep generative models.

Image based diagnosis plays are large part in every day clinical practice. Imaging equipment such as X-ray, computed tomography (CT), magnetic resonance imaging (MRI) and others provide large amounts of image data. Previously, this image data needed reviewing by human experts, such as radiologists to spot disease so that appropriate courses of suitable treatment action could be informed.

There is an ageing population in some parts of the world, putting a strain on national health care systems. Radiologists in (large) medical facilities are sometimes overloaded with images to be reviewed. Errors may crop in due to fatigue, which may have devastating consequences for patients.

Recently, computerized machine learning "(ML")-based systems have come into use to assist in reviewing medial imagery at thigh throughput. Such systems can help the radiologist in their work. Such machine learning based systems use certain algorithms that process previous data (training data) to learn diagnosing certain diseases. Before such ML systems can be put to clinical practice, they need to be suitably trained on a suitable body of training data. Training data may be labeled in some implementations. Labeled data may be obtained by extracting certain keywords from health records, reports, or other data associated with previous imagery of patients as held in medical storage facilities. As such, ML system for diagnostic use are medical devices and may need to be approved for such use by regulatory bodies.

Machine learning, that is the application of learning algorithms to training data is not necessarily a one-off, but may be done repeatedly over time as new data emerges. Such ML setups may be referred to as dynamic or adaptive ML systems.

Regulatory bodies like the FDA are beginning to consider adaptive machine learning as an integral part of medical devices. See for example the FDA discussion paper, "Proposed Regulatory Framework for Modifications to Artificial Intelligence/Machine Learning (AI/ML)-Based Software as a Medical Device (SaMD) - Discussion Paper and Request for Feedback", published April 2019, at the time of writing available online at https://www.fda.gov/media/122535/download.

In this new environment, adaptive machine learning models that employ additional data to improve/calibrate model performances may not require a new approval for each single model update but rather one approval for the adaptive learning framework itself.

In an adaptive learning system, the model should be able to identify the cases that it misclassifies or the cases it cannot handle with sufficient confidence and use them as additional training examples to improve model performance.

One challenge specifically for x-ray data is that there can be cases with high levels of uncertainty, but which are not appropriate for improving the performance of an ML model for certain pathology identifications.

SUMMARY OF THE INVENTION

There may therefore be a need to address at least some of the above-mentioned challenges, or to improve ML systems for use in the medical field more generally.

The present invention is defined by the independent claims where further embodiments are defined by the dependent claims.

It should be noted that the following described aspect of the invention equally applies to the to the method for providing training data, to the computer program element and to the computer readable medium.

A first aspect of the invention provides a system according to claim 1.

In embodiments, the input data is added to the said deployment experience set.

In embodiments, the analysis by the system is based on at least one criterion and wherein the criterion is adaptable based on the size of the deployment experience set.

In embodiments, a counter is configured to track per class the said size of a subset of said deployment experience set. The subset includes the counts for the respective class.

In embodiments, the said criterion is relaxed so as to increase the number of future input images indicatable as useful for retraining. The criterion may be threshold based. The threshold may be adapted to effect the relaxing.

In embodiments, the analysis by the system includes the system performing an outlier analysis in respect of the uncertainty value relative to uncertainty values of the previous input data for the said class.

In embodiments, the system is capable of identifying the input image, when found useful for retraining, to be representative of a new class, not among the pre-defined classes.

In embodiments, the identifying is based on an $n(n\geq2)$-dimensional outlier analysis of the of the uncertainty value relative to a latent space of the trained machine learning model for previous classifications. Here $n$ is a count of a number of classes; a number of images that were already assessed to be of high uncertainty; a number of region of images with high uncertainty scores; a respective proportion/frequency.

In embodiments, the decision logic is configured to trigger retraining by a training system of the trained machine learning model, based on a training data set including one or more such input images indicated as useful for retraining.

In embodiments, the retraining is so triggered if the number of such input images excessed a pre-determined threshold.

In embodiments, the trained machine learning model is a classifier of any one of the following types: artificial neural network model, support vector machine, decision tree, random forest, k-nearest neighbor, naive Bayes and linear discriminate analysis.

In embodiments, the input image is of any one of more of the following types: X-ray, magnetic resonance, ultrasound, nuclear.

In embodiments, the system may comprise the machine learning model and/or the training system.

Another aspect provides an arrangement comprising the system of any one of the above-mentioned embodiments, and i) an imaging apparatus supplying the input image, and/or ii) a memory from which the input image is receivable.

Yet another aspect provides a computer-implemented method according to claim 12.

Another aspect provides a computer program element, which, when being executed by at least one processing unit, is adapted to cause the processing unit to perform the method.

Another aspect provides at least one computer readable medium having stored thereon the program.

The deployment experience set represents all images that have been processed by the machine learning model or since the last training.

In embodiments an outlier detection approach is used, that has been found to yield good result for instances of abnormalities/diseases/conditions or other medical classes of interest that occur frequently enough during deployment. In addition, the system is capable of improving classification performance even for rare classes (e.g., rare disease), as the system processes ever more data during deployment. Ordinarily, because of their infrequent occurrence, rare abnormalities would be seen as kind of outliers, at least for some time. The issue of whether there are outliers is addressed in some embodiments of the proposed system by using a global occurrence counter per abnormality: if this counter is below a certain threshold (i.e. the system did not see enough of such abnormalities) the expectations of uncertainty score are adjusted (relaxed) so that the particular sample could still be added to the training set. The machine learning model of the system can then be retrained for classes of such rare abnormalities too. It is also envisaged herein to use such an approach in order to add new tasks (classes) to the model's reach. If a certain amount of samples with high uncertainty are observed, this may indicate a new finding(class). The experience set may thus be used to form a new dataset for training.

The proposed system and method address the challenge that in certain machine learning models, such as in artificial neural networks with more than one hidden layer ("Deep Learning") and others, the currently used image processing does not provide information about the boundaries of the class distributions that those networks are generally predicting. This makes it challenging for an automated system to infer, based on an input image, whether it can be a useful example to improve the performance of the model.

For x-ray classification, this problem cannot usually be handled by out-of-distribution models (like anomaly detection), because an x-ray image maybe of sufficiently good quality to diagnose certain conditions but not others. Moreover, the use of additional labels (beyond standard image interpretation), provided by radiologists, to classify whether an image is of good enough quality to diagnose a certain condition would be time consuming and limit the efficiency of such an in-product learning system (as opposed to for example automatically extracting image labels for radiologist reports).

The challenge when using the standard image-labels extracted from a radiology report is that absence of a medical term should not be interpreted as an absence of a specific condition. For example, in a given x-ray image, the mere absence of the term "cardiomegaly" in the radiology report may simply mean that this condition could not be assessed from the image due to image quality, or that the diagnostic purpose of acquiring the image was not related to the heart at all, or was related to other aspects thereof, etc.

The proposed system may be implemented as a software or hardware component (or as both).

The system and method as proposed herein is preferably used in the context of an in-product/dynamic learning system.

The proposed system determines the usefulness/harmfulness of a given image per class, such as disease/-pathology level. This allows to make more efficient use of the imagery encountered during deployment. For example a given image may have high uncertainty, or be not useful for training a model with a cardiomegaly class, but may be appropriate for improving atelectasis detection. The models used herein are preferably capable of computing the uncertainties independently for the different classes.

In some embodiments, the proposed system allows for the computation of two types of uncertainties per image, referred to herein as an alpha type uncertainty and a beta type uncertainty. The original uncertainty q may thus be resolved into two components: An alpha type uncertainty that quantifies the potential usefulness of the input image (for a given class) to improve the performance of the model by including it as additional training data. The beta type uncertainty quantifies the potential to reduce/harm the performance of the model (for a given class) if that image were to be included as additional training data for the given class. However, the alpha and beta uncertainties may not necessarily be computed explicitly herein, but may be used implicitly such as in a thresholding scheme as described herein. I other embodiments, there is no computation of quantification of the uncertainties.

In another aspect there is provided a computer program element, which, when being executed by at least one processing unit, is adapted to cause the processing unit to perform the method as per any one of the above-mentioned embodiments.

In another aspect still, there is provided a computer readable medium having stored thereon the program element.

The proposed system and method may be a used for transparent in-product machine learning systems. The system may be implemented in the imaging apparatus (X-ray, CT. MR, US etc.), or may be used in a diagnostic workstation or a cloud-based solution.

The in-product learning approach improves and fine-tunes abnormality classification models, in particular of radiology based diagnostic set-ups.

The transparent retraining approach for the ML model may obviate the need for recertification by regulators.

*"user"* relates to a person, such as medical personnel or clinical user. In other words, the user is in general not the patient.

In general, the term *"machine learning"* includes a computerized arrangement that implements a machine learning ("ML") algorithm. Some such ML algorithms operate to adjust a machine learning model that is configured to perform ("learn") a task. Other ML algorithms operate direct on training data, not necessarily using such a an (explicit) model, such as in clustering. This adjusting or updating of the model based on a training data corpus is called "training". In general task performance by the ML model may improve measurably, with training experience. Training experience may include suitable training data and exposure of the model to such data. Task performance may improve the better the data represents the task to be learned. *"Training experience helps improve performance if the training data well represents a distribution of examples over which the final system performance is measured"*. The performance may be measured by objective tests based on output produced by the model in response to feeding the model with test data. The performance may be defined in terms of a certain error rate to be achieved for the given test data. See for example, T. M. Mitchell, "Machine Learning", page 2, section 1.1, page 6 1.2.1, McGraw-Hill, 1997.

BRIEF DESCRIPTION OF THE DRAWINGS

Exemplary embodiments of the invention will now be described with reference to the following drawings, which, unless stated otherwise, are not to scale, wherein:

Fig. 1 shows a block diagram of an imaging arrangement;

Fig. 2 shows a block diagram of a machine learning based system for classification of imagery with dynamic learning;

Fig. 3 shows example imagery representative of different classification uncertainties;

Fig. 4 is an illustration of operation of the system in Fig. 2;

Fig. 5 shows a block diagram of a machine learning model;

Fig. 6 shows a block diagram of a training system for training a machine learning model;

Fig. 7 shows a schematic diagram of a workflow using the system of Fig. 2;

Fig. 8 is a flow chart of a machine learning based method with adaptive filtering for future training data; and

Fig. 9 is a flow chart of a method for training a machine learning model.

DETAILED DESCRIPTION OF EMBODIMENTS

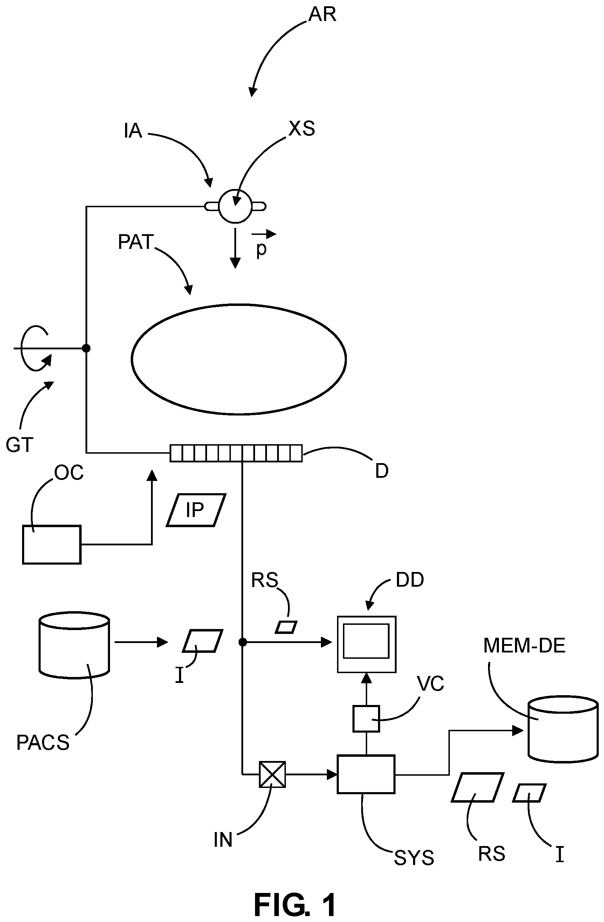

With reference to Fig. 1, this shows a block diagram of an arrangement for image processing. The arrangement includes an imaging apparatus IA (sometimes referred to herein as "the imager") and a computer-implemented system SYS. The imager supplies medical imagery of a patient. The computer-implemented system SYS process the imagery to support a clinical user. Preferably, the computer-implemented system SYS provides computer-assisted analysis of the imagery to produce a medical result, such as classification result. In preferred embodiments the system is based on machine learning and implements dynamic leaning where a machine learning model of the system is one or more times retrained on new imagery encountered during deployment. The system is capable of distinguishing which new imagery is useful or retraining and which is not, thus facilitating consistent and/or improved performance, with an ability of identifying training imagery suitable for training for hitherto not defined new classes. The system can thus be able to recognize even rare diseases or medical conditions and in an autonomous manner.

The imaging apparatus IA is preferably an X-ray imaging apparatus that produces a single ray exposure, such as in radiography, or, as in fluoroscopy or angiography applications, a sequence of X-ray exposures referred to herein as frames or images.

The imagery produced by the imager IA may be processed, possibly alongside other input, by the computer system SYS to compute a medical result RS to assist the clinical/medical user, such as a radiologist. Online and off-line embodiments of the system SYS are envisaged herein. Thus, the computer system may be a standalone system, not necessarily coupled to any imaging apparatus. That is, the processed imagery may not necessarily be "live" imagery, but may instead be historic imagery, retrieved from an image database or other storage, such as a PACS (Picture Archive Communication System) or other medial data repository.

Before explaining the machine learning based system SYS in more detail, the imaging procedure is briefly described first. Specifically, the imaging apparatus IA may be of the X-ray type, configured to acquire X-ray projection image(s). The image may be a (single) still image, such as in radiography, or may be part of a stream of images ("frames") that is used to display a video feed such as in fluoroscopy. The still image or stream may be displayed on a display device DD.

The imaging apparatus IA includes an X-ray source XS and an X-ray sensitive detector D. In particular in tomographic imaging, the imaging apparatus IA preferably allows acquiring projection imagery from different projection directions d. The imaging apparatus may include an optional rotational gantry GT to which the X-ray source XS and/or the detector D are connected. The projection imagery may be acquired along different projection directions by rotation of the gantry (and with it, of the source XS and optionally the detector D) around the lesioned site ST or ROI. Such gantry-based imaging apparatuses include C- or U-arm systems and are mainly envisaged herein, but so are (CT) computed tomography scanners. Non-gantry based imaging solutions are also envisaged, such as mobile or portable imaging devices, where there is no, or no permanent, physical connection between detector D and radiation source XS. Also, radiographic imaging setups are also envisaged herein instead of tomographic or rotational imaging (such as C-arm), in which case, again, no such rotatable gantry GT is required.

In more detail, during imaging, the X-ray source XS emits an X-ray beam which propagates along projection direction d to interact with patient tissue in the (current) field of view (FOV), to cause a modified X-ray beam to emerge at the far end of the patient, and be detected at detector D. Data acquisition circuitry (not shown) of the detector D converts the received modified radiation into a set of numbers ("pixel values"), preferably stored in a respective matrix per frame/image, with respective rows and columns. The rows and columns define a size of the image/frame. The pixel values represent detected intensities. The pixel values per frame/image can be used by a visualization component VC to effect display of the image on the display device DD during the intervention. In the following we will no longer distinguish between frame and still image, and simply use the term "image/imagery" as a generic reference to both.

Operation of the imaging apparatus, in particular image acquisition, is controlled by the user from an operator console or control unit OC. Operator console may be arranged as a dedicated computing unit communicatively coupled to the imaging apparatus. The operator console may be situated in the same room as the imager IA or in an adjacent "control room". Remotely controlled imaging systems IA connected through a suitable communication network to a control unit OC, located possibly remotely from the imaging apparatus, is envisaged herein in embodiments. Autonomous imaging system are also envisaged herein, where the control unit OC operates fully or semi-autonomously, without or with little user input. The imaging control unit OC controls the imaging operation by setting certain imaging parameters IP. The imaging parameters determine or at least influence the contrast mechanism and hence the image quality of the produced imagery to be processed by the system SYS. The intensity values (and hence the pixel values) as captured in the recorded imagery can be influenced by changing the imaging parameters.

The imagery *I* generated by the imager IA and to be processed by the system SYS may be projection imagery in projection domain as recorded by the detector D such as in radiography, or may comprise reconstructed cross-sectional imagery ("slices") in image domain obtained by a computed tomography algorithm.

Whilst main reference has been made to X-ray imaging, this is not at the exclusion of other imaging modalities. That is, the imaging apparatus IA may be an MRI imager, a PET/SPECT imager, an ultrasound imager, etc. Instead of CT, other tomographic imaging modalities such as OTS, etc are also envisaged.

The imagery acquired by the imaging apparatus IA includes image structures or feature(s). Image structure/-image feature is the variation of patterns of the pixel values in the captured imagery which may vary spatially across the image plane. Types of image structures may include geometrical structures or appearances at one or more scales, geometrical configuration (position, rotation, shear), gradients, color or grey value distribution, etc. In particular with X-ray imagery, such structures are a function of projection direction for a given object in 3D. Such image structures may be local or global.

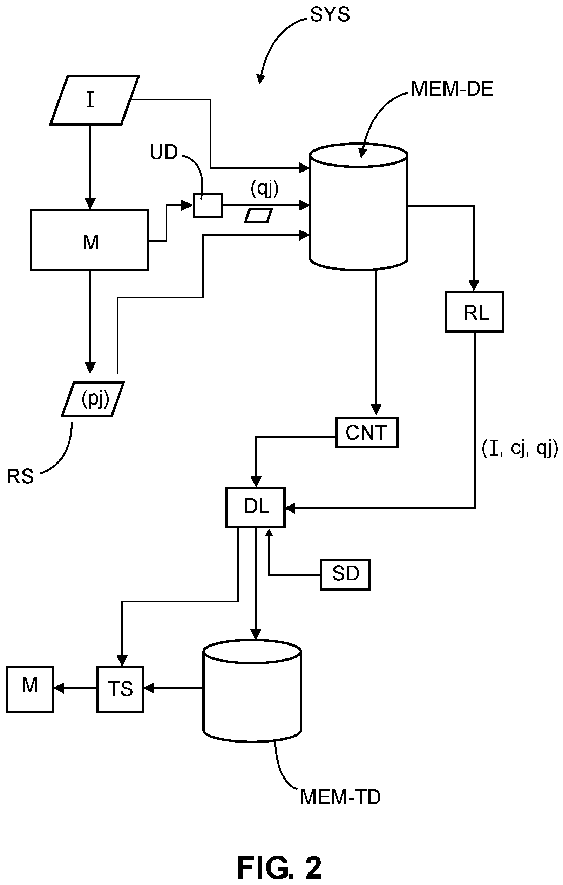

As briefly mentioned, the system SYS processes the imagery and produces as output the medical result RS. This is shown in the block diagram of Fig. 2 in more detail. The system SYS preferably uses a machine learning model M trained on training data. Specifically, and in some embodiments, the machine learning model M that has been trained on the training data. The training data includes training imagery. The training data may have been generated in historical interventions or exams or on other occasions, and may be held in medical data repositories such as a PACS HIS (Hospital Information System), patient health records, and so on.

Once trained, the model M can be used in deployment, such as in day-to-day clinical practice. The result RS produced by ML model M in deployment may be in text or image form, or in any other data type form, or in a combination thereof. The result RS produced by the machine learning based system SYS is to provide the user with information that can be gained from the imagery. As mentioned, in embodiments, in addition to the imagery, other (contextual) data may be co-processed with the imagery to arrive at the result RS. The result may be one of regression, or classification or any other, such as clustering, text generation. For example, the output result RS may be one of a diagnosis. Specifically, based on an image of a patient acquired by the (or other) imaging apparatus IA, a diagnosis is computed in respect of the patient. For example, a lesion is classified as benign or malign, etc. The system may be used in chest X-ray for example to classify a chest X-ray to be representative of any one of more medical conditions such as pneumothorax, cardiomegaly or other. Processing by the system SYS of imagery of other anatomies are also envisaged, such as brain scans, imagery of extremities such as arms and legs, abdominal imagery etc. Image-based recognition of individual organs, anatomies or parts thereof are examples of other such medical results RS envisaged herein.

In other words, the image arrangement adds value in providing additional information extracted from the imagery that may not be easily discernable by a human user. The medical user may inexperienced or may be in a situation of high stress as often happens in an operating theatre or a trauma room in a busy clinic for example. The image arrangement can thus support the medial user in image interpretation and/or diagnosis.

Referring now to Fig. 2, and in more detail to the machine learning system SYS, this includes in embodiments the pre-trained ML model M that has been adapted based on training data, such as historical patient imagery sourced from medical data repositories. The training data is held in on one or more training data memories MEM-TD. A training system TS, implemented on one or more computer systems, processes some or all of this training data as held in the training data base(s) MEM-TD. The training system TS adjusts parameters of the model M to so train the machine learning model M. Nonmodel based approaches are also envisaged herein, where input during deployment is adapted based on training data itself, to arrive at the result RS.

The performance of the computerized ML system SYS may depend on how well the machine learning MLM has been trained. The quality of training will depend, at least in part, on certain features of the training data set TD used. The training data set may comprise a large number, possibly, but not necessarily, in the order of hundreds or thousands, specimens of training imagery.

In machine learning, two processing phases may thus be distinguished herein: a training phase (also referred to herein as the learning phase), and a deployment phase. In training phase, the training system TS may use a training/learning algorithm to adjust parameters of the model M. The model is either an initial model one or a pre-trained one if, as envisaged herein, the training phase is to be repeated in cycles one or more times after an initial training phase. Some training algorithms can be formulated in terms of an optimization procedure where the parameters are adjusted so as to improve an objective function F, such as a cost function or utility function. Once sufficiently trained, for example once the cost function has dropped under a pre-defined acceptability criteria threshold, training can conclude at this cycle and the model may be released for deployment. In deployment, based on the training model, a prediction algorithm is used based on the model to compute, from a new image, a (medical) result RS of interest, such as a classification result. The image processed by the prediction algorithm in deployment is "new" in the sense that the training system TS has not been exposed to this image before, so did not form part of the previous training set which was used for in previous training cycle.

As will be discussed in more detail below, various training and prediction algorithms and, optionally, related ML models are envisaged herein. Such ML models may include artificial neural network ("NN") type models that may be trained based on gradient-based methods, such as backpropagation algorithms, etc. Machine learning set-ups envisaged herein can be of the supervised or unsupervised type. In supervised learning, the training data is labeled. The label for each training image represents a pre-assigned class based on prior clinical domain knowledge. A human expert for example may review historical (that is, existing) patient imagery and assign suitable labels for each. The labels may be automatically recovered by a scripting tool for example, configured to automatically search through radiological or other expert reports stored in a medical database, in association with the respective historical imagery to which the respective report pertains. The scripting or other such database searching tools, may use natural language processing (NLP) tools to identify certain tell-tale key words in reports associated with the stored imagery, to so attach a label to the historical image. In this manner the training data set S can be built up and stored in a memory MEM-TD for used in the training phase.

Examples for such data storages from which a training data set may be sourced and compiled are medical patient data storage facilities, such as a PACS (picture archiving and communication system). Sometimes, depending on the storage format used, the keywords may be extracted in metadata of the imager, such as header data. The DICOM format for example may allow for such metadata. The result of manual or such automated training data sourcing is the said training data set $S = \{(x_f, y_f)$, with $x_j$ a training image and $y_j$ its label (or target). The label may $y_j$ may be a code or symbol, string, etc. for a medical condition or disease that a radiologist or other expert (who drew up the report) believes presents in the patient as per the image $x_j$.

The ML models mainly envisaged herein are of the classifier type as indicated above. The models, once sufficiently trained, are capable of classifying an image encountered during deployment into any one of a number of pre-defined classes. Each class is representative of a medical condition, for example a disease or similar. Whilst a single class may be sufficient for some purposes, it is mainly multiclassifiers that operate to classify imagery into one (or more) of plural classes that are envisaged herein. For example, certain imagery, such as chest imagery, may be used to diagnose a number of different diseases. Each disease may be represented by a different class. In more detail, chest ray imaging may encounter heart or lung disease and the system may be able to classify the imagery accordingly. It is thus thought that, given the image, the classification score in general correlates with a reason to belief that the patients presents with the respective disease. Further medical analysis or medical steps (exams, etc.) may then be scheduled for the patient accordingly.

The output (the result RS) of the classifier machine learning model M may thus be understood as a vector with entries that may represent respective classification scores $p = (p_i)$ for the respective class $i$ or disease. For example, for mutually exclusive classes, a soft-max layer may be used in an NN-type model as an output layer OL. Unlike the input or one or more hidden layers, the output layer is capable of "within"-layer normalization to furnish such a probability vector.

In the following we will mainly refer simply to classes $C_j$, with the understanding that each class represents a respective disease, condition, or other medical category. However, in some embodiments it may also be advisable to use a separate class, a place-holder-class C* as it were, for an as yet unknown or unexpected disease/category that may be found during the deployment phase as will be detailed below. The classification scores $p = (p_i)$ are not necessarily probabilities, but may relate instead to more general scores that are thought to correlate as mentioned above. Whilst the range of some or each of the values $p_i$ may be in the unit interval, the scores, when summed over class $i$, do not generally yield unity. Also, whilst multi-class classification with single label per class may be envisaged in some applications, this is not necessarily required herein, and multi-label classifications are also envisaged herein to support diagnostics of comorbidities. That is, the classification scores $p$, even if interpreted as probabilities, may still not necessarily add up to unity because of the said co-morbidities. A given image may thus be classified into more than one class.

As preferred herein, some ML setups may include an uncertainty determiner component UD. Such ML setups provide, in addition to the described classification score vector, related uncertainty values $q=(q_i)$, each $q_i$ associated with a respective one of the classification score $p_i$ for a given class $i$. Thus, in such machine learning set-ups mainly envisaged, the output of the machine learning model may be written as $p = ( (p_i, q_i) )$. The uncertainty value $q$ may be computed by mapping into a finite interval, such as a unit interval [0,1], but this is not a necessity herein and any other interval suitable for the task at hand may be used instead. A reference herein "$q$" is a generic shorthand reference to any given uncertainty values for any given class $I$, or to a subset or all of the q's collectively.

The uncertainty value quantifies a respective uncertainty that attaches to the respective probability $p_j$ for class $C_j$. For example, the probability for belonging to class $i$ may be 80%, and there is a *10%* uncertainty that this result, $p_i = 80\%$, is true. The uncertainty determiner UD may be provided as an additional processing component or may be integrated into the model M. For example, a Bayesian framework may be envisaged herein, p and/or q may be understood as a measure for a degree of belief, given all the information available at the time of the prediction $M^\theta(I) = p$, including the (deployment) input image $I$, and the information encoded by the trained model M. The notation $"M^\theta(I)"$ is shorthand for the model M, with parameters $\theta$ learned in the previous learning cycle, applied to current deployment image specimen $I$.

The uncertainty may be further understood to arise in the specific field of image-based medical diagnostics mainly envisaged herein. The model M is usually trained using mainly (if not exclusively) information about the presence of a specific class, and not necessarily the absence of it. This situation arises frequently in radiology for example, where a report contains a list of findings and only in some cases a confirmation that certain pathology is not present (for example, a statement such as "no pneumothorax"). This means that out of the three possible scenarios, "pneumothorax present", "pneumothorax not present", "not possible to deduce based on the image", the model M mainly encounters imagery with positive examples, such as where pneumothorax is present. The uncertainty quantity $q$ as computed herein is then able to differentiate between the scenarios "pneumothorax not present" and "not possible to deduce based on the image".

Generally, one may distinguish herein between two types of uncertainties q, aleatoric and epistemic. Aleatoric uncertainties may stem from deficiencies in the image acquisition procedure that may result in poor imagery. Such imagery may be poor in the sense that it may occlude, cloud or otherwise fail to capture some features essential for the medical task at hand, thus causing uncertainty in the classification. For example, wrong or sub-optimal imaging parameters may have been used in error by an inexperienced medical user. For example, the image may be overexposed, or the relevant anatomy is at least partly out of the field of view as may happen by incorrect collimation, etc. More generally, aleatoric uncertainty reflects the noise inherent in the observation, which could result from imaging deficiencies as mentioned above, but may have other cases.

Epistemic uncertainty relates to the fact of a poor training experience of the model. Epistemic uncertainty stems from a lack of "knowledge". That is, the trained machine learning model at its current training stage had not been exposed to a sufficiently large number of relevant case imagery for a given disease so as to be able to make predictions with sufficient certainty. Said differently, the training data set S on which the model has been trained did not represent well the distribution for some or all classes of interest.

That is, the uncertainty value(s) $q$ computed herein may refer to epistemic or aleatoric uncertainty, depending on the underlying uncertainty determination algorithm run by the uncertainty determiner UD. A dual channel uncertainty determiner is also envisaged in some embodiment where for some or each prediction value $p$, both types of uncertainties, aleatoric $q^a$ and $q^e$, may be computed herein. However, computing merely one type or uncertainty values, $q^a$ or $q^e$, but not both, is mainly envisaged herein. A generic reference herein to uncertainty value q is thus a reference to either one of $q^a$ and $q^e$. In some cases, a given uncertainty value q may be thought of including contributions from both types of uncertainties. The uncertainty values may thus be written as a, in general unknown, function $H$ of an aleatoric and an epistemic uncertainty component, $q = H(q^a, q^e)$.

The imagery processed by the trained model in deployment during clinical practice over a certain period of time represents a valuable source of additional training data. This additional training data may be used to re-train the model in the proposed dynamic learning scheme in a next training cycle. This re-training or next training cycle may be triggered after a sufficient number of new training data have been processed by the model M. The set of imagery processed (classified), by the model M up to a certain period of time after the last training cycle, may be referred to herein as the deployment experience set $S'$, which may be stored in a non-volatile memory MEM-DE. The retraining may thus be triggered once set S' has grown to a certain size. Alternatively, or in addition, a statistical analysis may be performed on the imagery classified thus far in order to ensure that imagery from patients with sufficiently varying demographics has accumulated in set S'.

It has been found that not every (new) image in S' may necessarily improve the performance of the machine learning model. For example, poor imagery may lead to high aleatoric uncertainty, and use of such imagery as training data may actually compromise the performance of the model M.

The proposed system SYS acts thus as a filter to analyze the imagery accumulated in the deployment experience set S'. The system proposed herein acts as a filter to filter-out images that are harmful to the performance of model M and should not be used for training. In addition or instead, images in S' that are useful for re-training are identified. The system SYS distinguishes those images in set S' that are useful and should be used for training, as such imagery is likely to improve performance of the model M. As used herein, an image in deployment experience set S' is "useful" if its inclusion into a training cycle leads, or is likely to lead, to an improvement of model performance. Otherwise, the image is called (potentially) not useful/ useless/harmful. Model performance may be measured on test data with test scores assigned based on the proportion of correctly classified test cases.

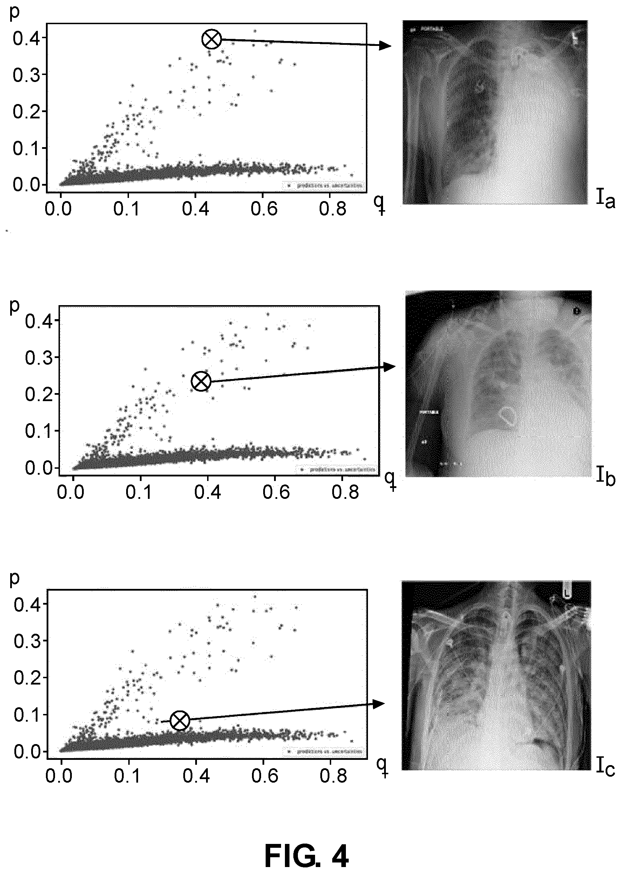

In addition, in a refined embodiment, and as will be explained in more detail below, the system is envisaged in some embodiments for refined processing in that it further distinguishes, among the images that have been found (potentially) harmful, those that are genuinely harmful from those that are potentially still useful for training as such images may relate to rare diseases. The system may thus implement a two-tier rejection scheme, where there is no blanket rejection of images that have been found potentially harmful as these may still be admitted. The system is thus configured to be sensitive to imagery that pertains to rare cases to so ensure such imagery is still included in the training. Specifically, the system SYS may keep track of the potential similarities between the images that are found to be of harmful. A large concentration/cluster of such similar images (in a certain data space, referred to herein as the $(p,q)$-space, on which more below at Fig. 4), above a certain threshold, may indicate a rare disease, rather than a deficient medical image. Image similarity may be measured by any suitable metric, (squared) Euclidean distance, or other. If a concentration of such images above a certain threshold is detected, these can be reviewed and added as a new class label if needed. Such rare disease may already be known and there may already exist a specific corresponding classification class in the model.

Alternatively, the earlier mentioned generic place-holder class may be used to capture such rare diseases, yet unknown. The system may recommend defining a new class to so enrich the current model.

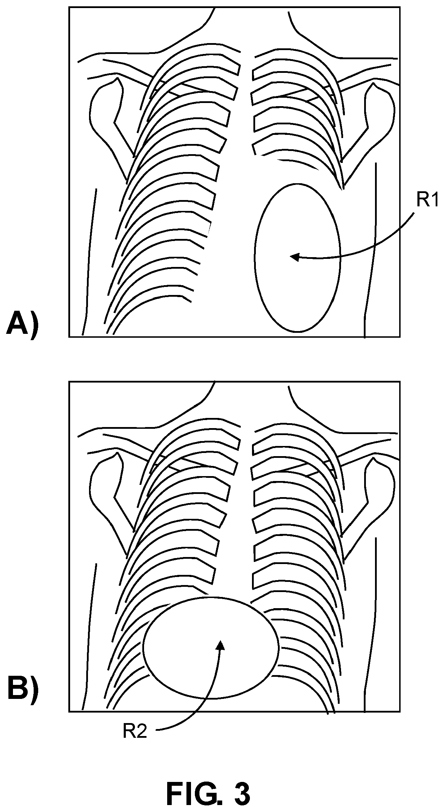

Fig. 3 shows examples of imagery that may be useful or harmful for training. In more detail, pane A) shows an example X-ray that is likely to be harmful for cardiomegaly classification (a specific class) since the heart is not visible in this image. Pane B,) illustrates an example of potentially useful imagery. Thus, more images of similar orientation and quality may be useful to improve the performance of the model for cardiomegaly classification. Regions R1,R2 in Fig. 3 represents image portions that attract high levels of uncertainty , as medically significant image information is drowned out by overexposure artifacts for example. However, in pane B the occlusion R2 does not disturb cardiomegaly classification for example, whilst occlusion R1 in pane A does. In case of mis-positioning of the patient or mis-orientation, the region R1 can be thought of as circumscribing the whole chest area. Thus, as illustrated in Fig. 3, for certain pathologies, an image may not be useful for identifying certain pathologies (ie, the heart in an x-ray may not be visible, so cardiomegaly cannot be assessed), but may be useful training material for other pathologies that can be identified. In such situations, if the classification model M is of the neural network type for example, there may be certain activation region in the neural network model M, that are shared among all classes, and some parts that are separate. Retraining could be then targeted only for the cases where the image is helpful.

As proposed herein, in dynamic or in-product learning, the computed uncertainty value may be used to identify whether an image should be used to further improve the quality of the model and for which condition/class $i$ and/or which image should not be used for re-training.

Specifically, the system determines the image usefulness or harmfulness based on the $q$ value of the current image. The system is configured to compute an indication on potential usefulness of the input image (for each or some condition $i$ of interest) to improve the performance of the machine learning model by including this image as additional training data if found useful. In addition or instead, the system may compute an indication for the current image to potentially reduce/harm the performance of the model (for some or each condition of interest) if it were to be included as additional training data for a the respective condition of interest.

With continued reference to Fig. 2, and in more detail, the machine learning model MLM, of system SYS includes the machine learning model M. The model M was trained in a previous cycle on training data. The training data is stored in a training data memory MEM-TD. In deployment, the model M processes a current image $I$ to classify same into respective classes, to produce a probability vector $p=(p_j)$ indicative of a medical result RS. For example, one such class may correspond to cardiomegaly for chest X-ray. It is this classification result/score $p=(p_j)$ that may be displayed graphically to the user on a display device by operation of a visualizer VZ. Thus, the score vector $p$ may be displayed. As mentioned earlier, the score vector is not in general a probability distribution as the sum of $p_i$'s may not necessarily add up to unity as the classes are not generally mutually exclusive because the patient may present with more than one class of disease at the same time. Alternatively, and for example for such mutually exclusive classes, it is only the class that attracted the highest probability that is indicated on the screen, as desired.

The classification result $p=(p_j)$ (the classification score vector), may be stored in the deployment experience memory MEM-DE alongside the respective input image $I$. In addition, the uncertainty determiner UD determines an associated uncertainty $q_j$ which is likewise stored in the said deployment experience memory MEM-DE or elsewhere. The deployment experience set S's may hence be written as $S' = \{p^i =(p_j), q'=(q_j), I_j\}$. We will be using the generic notation "$q$" and "$p$" herein to refer generically to an uncertainty value or classification score or to a set of such value collectively. The reference "$p$-value/$p$ value" or "$q$-value/$q$ value" may also be used herein.

Explaining now operation of system SYS in in more detail, machine learning model M processes the current input image $I$ to compute the classification score p, The uncertainty determiner UD computes uncertainties $q$ for classification score $p$. A decision logic DL determines based on $q$ whether the current image is useful or harmful. If found useful, image is added to the training data set to be used for the next-retraining cycle of model M. If found harmful, the current image is not so added. The determination by decision logic DL on whether or not image is useful is computed based on the uncertainty value $q$ assigned to that image and the $q$ values of the previously classified images already present in the deployment experience at S'. A suitably configured measure Q may be used that quantifies how the current $q$ deviates from the earlier q's. As will be described in more detail a suitably configured thresholding may be used. It is thus assumed herein that the deployment experience sets already includes a sufficiently large number of classified images, that is, images classified after the last training cycle. In order to use the proposed training data filter system SYS, there may thus be a certain break-in period required where classified imagery allowed to accumulate firs in the data experience set.

Preferably it is only once a sufficient number of such data has been accumulated in S', that the system SYS becomes fully operational to decide whether to admit the input image for retraining. Again, as previously with the training data set, a statistical demographic analysis may to ensure that the number of classified images in the deployment data set is sufficiently varied. Thus, it may not necessarily be a fixed number threshold of required cases presently stored in the deployment data set that triggers the operation of the decision logic, but the demographic composition of the analyzed cases.

The processing by the decision logic DL proceeds per class $i$. In other words, the DL operates to determine whether the image is useful as a potential training image for the given class i. Optionally, the harmfulness of the image may be so determined. Thus, a certain input image may be useful for a certain (one or more) class, but harmful for other(s) class(es).

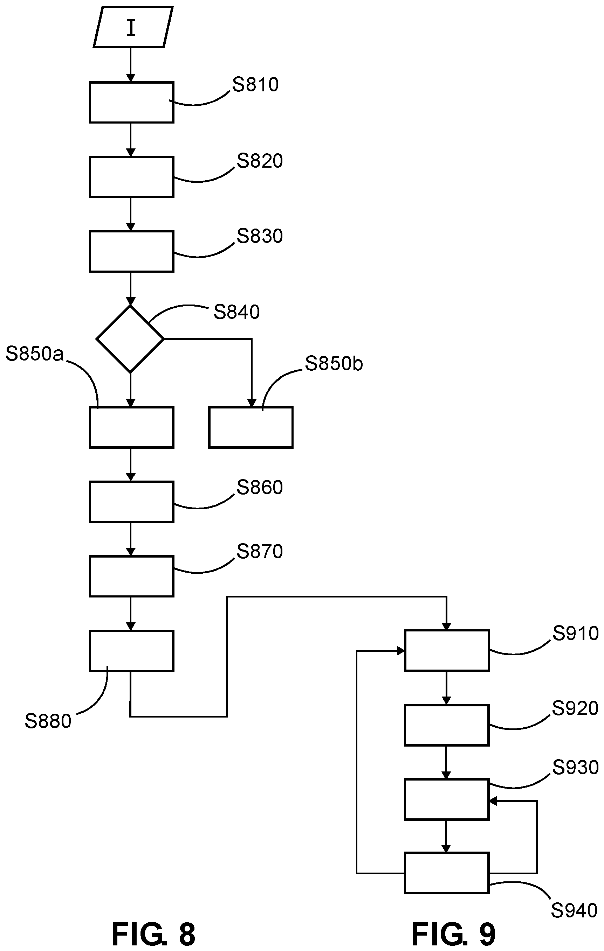

For a given input image, the decision logic DL may generate a set of indications for usefulness/harmfulness for some of each class. The indication may be binary (eg, coded as "1" for useful, and "0" for harmful) or as a usefulness/harmfulness score in a range may be computed instead. Such quantifications of the usefulness /harmfulness will be explained in more details below in terms of alpha and better scores at Fig. 8 below.

Optionally, and preferably for multi-label classification, a re-labeler RL may analyze the usefulness/harmfulness determination, to remove the respective label for which the image was found harmful. This relabeling operation by relabeled RL may ensure that each input image is used for future training only with a label for which it was found useful. In this manner, performance of the machine learning model in the next training cycle may be improved.

The decision logic DL (or a different logic) may monitor the number of cases flagged up for admittance into the next training cycle and storage in the training data set. Once a sufficient number of such admitted images (possibly re-labeled) has accumulated, that is, exceeds a pre-defined threshold, the next iteration learning cycle may be triggered and flow control passes to the training system TS. Again, as before, when triggering retraining, the underlying demographics of the new cases may be considered in addition to case number to ensure a sufficiently varied number of new training cases is admitted. Automatic triggering of re-training described is indeed in envisaged in embodiments, but so are embodiments where user is informed by display, sound, or other that a new training cycle may be run. It is then the user that manually authorizes such retraining, possibly after review of some of the new cases that have accumulated.

Training system TS use in particular the newly admitted training images (possibly with labels awarded by re-labeler RL) to re-train the model M. In retraining, the newly admitted cases are accessed and fed as training input data in the training system. The implemented training algorithm then uses their targets to further adjust the current parameters $\theta$ of model M to obtain an improved parameterization $\theta'$. The model M thus undergoes an enriched training experience and thus likely improves performance over a broader range of cases. It will be understood that the newly added training data is preferably used together with the previous training data to train the model. Training batches may be formed including a mix of earlier $(x,y)$ and newly admitted $(x',y')$ training cases.

An optional counter component CNT may be used that keeps count of respective sub-sets of the deployment experience training data set S'. Each such sub-set represents the number of specimens in the respective class. The counter CNT preferably operates according to the above-mentioned restricted mode and counts are awarded only for classes that attract the highest classification probability $p_j$ or for classes that attract probabilities beyond the significance threshold. The counts may be tallied as absolute counts or may be tallied as normalized counts (count rates) over cases processed. The counter may be reset for each training cycle or may be maintained over multiple cycles or over the envisaged lifetime of system SYS.

As will be explained in more detail below, the counter keeps track of the number of cases $l$ classified into the respective classes. The counts for each class may affect the criteria used by logic DL to determine usefulness/harmfulness for the respective class. Adaptive thresholding may be used by the DL. The per class threshold may be relaxed if for a given class, possibly representing a rare disease/category for example, only very few cases have been seen so far. The system is thus more likely to admit a given image as useful for training for a low class with low count, as compared to a class where more specimens have been seen. The system is hence adaptive in that different criteria are used by DL for each class to determine usefulness/harmfulness.

The system is adaptive and self-improving. The system SYS can improve its performance over time, even for classes that represent rare diseases. The overall distribution of diseases/medical conditions encountered in a given patient population may depend on the characteristics of this population. For example, in some regions of a country, or indeed in different countries, the prevalent conditions may differ. What is rare in one country or region may be quite prevalent in another. The proposed system with self-adaptive dynamic learning decision support based on data filtering described above can hence be used for different demographics. In other words, the very same system set-up may be used across highly heterogeneous geographies and yet the system, because by virtue of its self-adaptation, tends to compile a well distributed training data set in the different cycles that best represents the distribution of diseases in the respective geographies. International or regional deployment costs and efforts can thus be reduced. Also, repeated market authorizations awarded by medical regulators may not be necessary, or at least accelerated authorization-tracks may be available for each training cycle.

Operation of the decision logic DL may be illustrated with reference to the diagrams A-C as shown in Fig. 4. The figure illustrates $(p\text{-}q)$ diagrams where the respective p,q values for a given class are represented as points in the $(p,q)$-plane. As before, p represents the probability for the respective class computed by ML model M, and q is the associated uncertainty. Each point in diagram A-C thus represents the respective $(p,q)$ value computed for given class for images in the deployment experience data set.

As can be seen, point clouds tend to form, with some clustering definition in the (p,q)-plane. Some exemplary images are indicated with a respective cross 'X' in the diagram, shown in panes A, B, C. The diagrams A-C are identical but each focuses on a different one of the images. shown by the respective cross. The crossed-images are examples of cases that present with relatively high uncertainty, as compared to the uncertainties of the previously classified imagery. The "crossed"-cases or similar may be understood as outliers relative to the cluster.

More specifically, the crossed-images in diagrams A, B represent uncertainties as outliers in the (p,q) plane. In this examples, the crossed images appear as outliers in the (p,q) plane for the cardiomegaly class of the predefined classification classes $C_j$ for which model M is stet-up. The benefit of these samples for a re-training is therefore questionable and representative of harmful imagery. Their uncertainty values, when considered against the bulk of the q's in the experience set $S$', could therefore be used to remove those samples from the dataset, or at least the system may issue a signa to query a radiologist/domain expert for feedback (e.g. as a quality assurance measure).

The situation is different for diagram C in the bottom of Fig. 4. The image crossed in diagram is representative of a useful image: this time, the crossed images is still an outlier, but a "close" one as opposed to the "far" outliers as illustrated in diagrams A, B. The close outlier is located closer to an average uncertainty (represented by the cluster) for the specific prediction class. Such images with close outlier profile can represent cases with slightly different orientation or image quality as compared to what has been used to train the model, thus their inclusion, as additional training data, can improve the performance of the model.

In some embodiments, and turning now in yet more detail to operation of decision logic DL, an outlier analysis may be performed by decision logic DL. For each given q value for input image in given class, the decision logic compares the current q value against the previous (historic) q values of the previously classified images in the deployment data set S' as this point. The distance measure may be configured to measure the distance of the current q for a current image to the point cloud or cluster of *q*-values of the previously classified imagery.

For example, a statistical descriptor may be used to define measure $\mathbb{Q}$ such as a percentile, median, average or medium value that quantifies the collective of the previously collected q's. The descriptor may be configured to define a range of values. For example, the descriptor may be used to define a statistical threshold for the cluster. The current q value is measured against this threshold to determine usefulness/harmfulness of the associated current input image. For example, a current *q* value below the threshold are close outliers, representative of a useful image. But a current *q* beyond the threshold may be representative of a harmful image. The clusters may not always be apparent as in the examples shown in Fig. 4.

The measure Q can be adapted by factoring in the count rate provided by counter CNT for the given class. Moreover, CNT provides counts for similar images that have been found to be harmful.

The counter CNT thus facilitates identifying regions within the images that may be harmful. And the thresholding or other $\mathbb{Q}$ -measure may then be applied at the level corresponding to the similar images. Image similarity may be determined using any suitable metric, such as squared Euclidean distance, cross-correlation, or other template matching techniques. Alternatively, image descriptor-based algorithms such as SIFT, SURF etc. may be used. Machine learning based similarity determiner are also envisaged, such as CNNs or others. The similarity determination may be implemented by a (image) similarity determiner SD.

As mentioned above, the threshold and/or distance measure for each class *i* may depend on the number of cases as tallied by counter CNT and as previously assigned to this class to so account for rare disease, as fewer specimens are thought to be representative of such rare diseases. The above-described threshold for a give class defines an admittance criterion for use a training data and this may thus be relaxed over deployment time for each class depending on counter rate. The lower the count the mor relaxed the thresholding, and the stricter as the class count grows. This relaxation regime can be implemented by including a decay term into the distance-based thresholding described above to modulate the thresholding for such classes and their respective counts accordingly. For example, and depending on how thresholds are coded and which sense, the threshold may be higher the smaller the case count, and threshold decreases over deployment time as class count increases.

In general, a relatively high q value should be indicative of an outlier. Thus, a thresholding should be sufficient in most of the cases to establish what is an outlier instance for q. The exact threshold may be based on expert choice, or using a heuristic, eg, by taking a histogram of the q distribution into account as per the current experience set S'. Since there seems to be sometimes a correlation between *q* and *p,* the configuration of the threshold may be done in relation to *p,* e.g. by using a suitable binning of *p.* In general, cases with a high or low *p* value and a high *q* case is preferably not flagged up as an outliers.

In yet more detail and in embodiments, a class specific "outlyingness" function O() may be configured as the above mentioned descriptor. Outlyingness functions were described by M. Hubert et al in "Outlier detection for skewed data", published in Journal of Chemometrics, vol 22(3), pp 235-246, published March 2018.

In some embodiments envisaged herein, the outlyingness function $O^{k(q,Q)}$ may be defined sectionwise as follows:

$$O = 0: \text{if } q < median(Q) \tag{1a}$$

$$O = (q - median(\mathbf{Q})) / (w - median(Q)): \text{if } q > median(Q) \tag{1b}$$

(1a) ensures that cases with very low q are not considered outliers, whilst cases with high q are flagged as outliers. The value of O measures the outlyingness. Instead of the median-operator, any other statistical characterization such as averages, percentiles, etc. may be used instead. Characterizations in terms of higher moments, such as variances/Standard deviation are also envisaged herein.

In (1a,b) above, $Q$ is the set of uncertainty values of cases in set S', and $w$ is a normalization factor. Alternative approaches could include percentiles, or the fitting of density functions to the data.

Since there seems to be a correlation between $q$ and $p$, function $O$ may be restricted to all those $q$'s for which $p$ is in some interval $[p-\varepsilon, p+\varepsilon]$, with "$\varepsilon$" being a design parameter.

The dynamic, class specific threshold may be denoted as $T^{k(n)}$. The threshold is configured to change, depending on the counts tallied by counter CNT for the specific class:

$$T^{k(n)} = T_{init} / (1 + k * n) \tag{2}$$

with $k$ a decay term, and $n$ the counts for the respective class. Instead of, or in addition, $n$ may relate to the number of images that were already assessed to be of high uncertainty. $n$ may relate to the number of region of images with high uncertainty scores. Instead of absolute number counts, $n$ may relate to the respective proportion/frequency. Instead of measuring relative to $q$ as in (1) or (2), the beta (beta) component of the uncertainty $q$ may be used.

The decay term $k$ is configured to respond to the counts in the respective class to change the threshold as the number of counts in the given class increases. The thresholding (2) is thus modulated by the number of cases classified in the respective class. A separate and independent such modulation is done for some or each other class and the outlier analysis based on the respectively adapted threshold is done of each class separately. The threshold may drop with growing counts n as implemented in the example (2). An exponential decay term $1/e^n$ may be used instead.

It will be understood that the formulations at (1a,b) and (2) are, whilst envisaged in embodiments, exemplary and other outlyingness definitions and threshold modulations are also envisaged herein. Preferably, the adaptive thresholding used for beta scores (see below).

In general, as the system SYS evolves over time, that is, the more cases are processed by the model M and the decision logic DL, the more "confident" the system becomes. So, especially in an early phase with few case counts, the usefulness of high $q$ cases is less apparent, and a more relaxed thresholding (threshold high) is preferred. The thresholding becomes stricter (threshold drops) with growing case count.

The above-described approach with adaptive thresholding based on relaxing with increased class count, may also be used to add a new task/class for the model to the classify for. For example, if a large amount of samples with a high uncertainty are observed this may indicate presence of a new class (eg, a not envisaged abnormality, disease or condition, or indeed a not known condition/disease). The related images may thus be used to form a new dataset for training for a new class. Specifically, the thresholding was described above in particular in connection with Fig. 4, as a 1-D outlier analysis on the output q of the model M to identify images that are not useful to further re-train the model for a specific class. In order to find new classes, for example for a rare disease, not initially envisaged by the model, a similar outlier analysis may be done, but in 2-D or higher dimensions. In such an approach, the uncertainty values q may be related to parameters of the model M itself. For example, in the context of NN type models, an outlier analysis may be based on latent spaces of the model associated with the different classes of the model. Specifically, uncertainty q may be computed, not on the (1-D) output of the model (the classification result $p_i$ for a class $i$), but for the latent space. The latent space includes the parameters of a hidden layer of the NN model. This latent space may have dimension 2 or higher, depending on implementation. New images $I$ may cause outliers in this 2-D latent space initially, but after a certain period of deployment, if a number of such outliers are located in the same region of latent space, they can be considered as a potential new class $C^*$ that may be used to update the model M for classes $C' = (C_1, ... C_N, C^*)$.

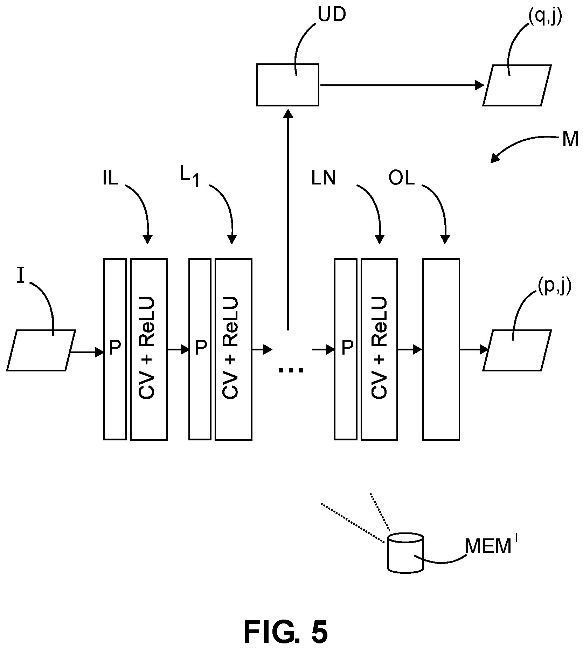

Reference is now made to Fig. 5 which shows an exemplary machine learning model M, as may be used herein in embodiments. The model may be stored in a memory MEM'. The model may be one of the NN-type. It may comprise computational nodes with adjustable weights $\theta$. The nodes may be arranged in a cascaded manner in layers, with input layer IL and output layer OL, and one or more hidden layers Lj in between. The output layer OL may be a soft-may layer to implement classification and to compute probability vector $p = (p_j)$.

The model network M may be said to have a deep architecture because it has more than one hidden layer. In a feed-forward network, the "depth" is the number of hidden layers between input layer IL and output layer OL, whilst in recurrent networks the depth is the number of hidden layers, times the number of passes.

The layers of the network, and indeed the input and output imagery, and the input and output between hidden layers (referred to herein as feature maps), can be represented as two or higher dimensional matrices ("tensors") for computational and memory allocation efficiency. The dimension and the number of entries represent the above-mentioned size.

Preferably, the hidden layers include a sequence of convolutional layers, represented herein as layers $L_1$ - $L_N$. The number of convolutional layers is at least one, such as 2-5, or any other number. The number may run into double-digit figures.

In embodiments, downstream of the sequence of convolutional layers there may be one or more fully connected layers FC, in particular if a classification result is sought, with the final layer OL combing the features maps into the probability score vector. A soft-max layer may be used in the final layer. The *softmax*-function layer OL or a similar computational node combines where feature maps from previous layer(s) into normalized counts to represent the classification probability per class for mutually exclusive classes. In cases where the multiple labels are not mutually exclusive (ie a person may have multiple pathologies that are visible in an x-ray) as mainly envisaged herein, instead of the soft-max function, a sigmoid type output layer is used.

Parameters of the model M to be adapted in the training includes weights of the layers. Preferably, some or all of the layers are convolutional layers, that is, include one or more convolutional operators/filters which process an input feature map from an earlier layer into intermediate output, sometimes referred to as logits. Using a convolutional architecture is advantages when processing locally correlated data such as images mainly envisaged herein. An optional bias term may be applied by in addition to the convolution filters. An activation layer processes in a non-linear manner the logits into a next generation feature map which is then output and passed as input to the next layer, and so forth. The activation layer may be implemented as a rectified linear unit RELU as shown, or as a *soft-max*-function, a sigmoid-function, *tanh*-function or any other suitable non-linear function. Optionally, there may be other functional layers such as pooling layers or drop-out layers to foster more robust learning. The pooling layers reduce dimension of output whilst drop-out layer sever connections between node from different layers.

Each hidden $L_m$ layer and the input layer IL implements one or more convolutional operators. Each layer $L_m$ may implement the same number of convolution operators CV or the number may differ for some or all layers.

The neural network model may be partly or wholly recurrent or partly or wholly feedforward. Non-neural network type models are also envisaged herein, such as support vector machines (SVM), *k*-nearest neighbors' methodology, decision trees, random forest, multivariate regression (weighted linear or logistic regression). Still other techniques may include Bayesian networks, or random fields, such as Markov type random field and others still that are based on training data.

The uncertainty determiner UD may compute the uncertainty value q, based on feature maps at a given depth. Operation of the uncertainty determiner UD may be based on Bayesian analysis or non-Bayesian statistics across the nodes of the model. In addition, to assigning weights (that is a respective number) to the nodes of the NN model as is customary done, a family of parameterized probability distributions/densities is associated to each node. The learning algorithm in the optimization adapts the parameters of the probability distribution for each node, and the weight of each node is then distributed as per the respective probability distribution. Standard deviations or variances may be computed for the probability distributions, suitably scaled and mapped into a range of values to compute the uncertainties q.

The computed classification probabilities may constitute priors in the Bayesian sense, thus allowing to compute the uncertainty values using the Bayesian formula. Bayesian analysis-based computing of uncertainty values may be based on drop-out layers, for example as described by Y Gal et al in "Dropout as a Bayesian Approximation: Representing Model Uncertainty in Deep Learning", available online at arXiv:1506.02142 [stat.ML], submitted 4 October 2016. A Kendall et al describe in "What Uncertainties Do We Need in Bayesian Deep Learning for Computer Vision?", available at arXiv:1703.04977 [cs.CV], submitted 5 Oct 2017, further approaches of Bayesian based uncertainty computations, also envisaged herein. However, the present disclosure is not confined to NN-type models. In particular, uncertainties q may be computed for other types of ML models, such as random forest classifiers (also envisaged herein) as described for instance by M H Shaker et al in "Aleatoric and Epistemic Uncertainty with Random Forests", available online on arXiv:2001.00893, submitted 03 January 2020.

Any other machine learning model setup than artificial neural network or random forests are also envisaged herein, such as convolutional neural networks, support vector machines, decisions trees, k-nearest neighbor, naive Bayes, linear discriminate analysis as well as ensemble and boosting techniques. Another approach to estimate uncertainty measure q is to computing multiple ML models, and to determine the statical variance in the respective predictions.

The rareness of a class may be computed by a similar outlier analysis of q relative to the parameters of a given hidden layer for example in relation to neural network type models.

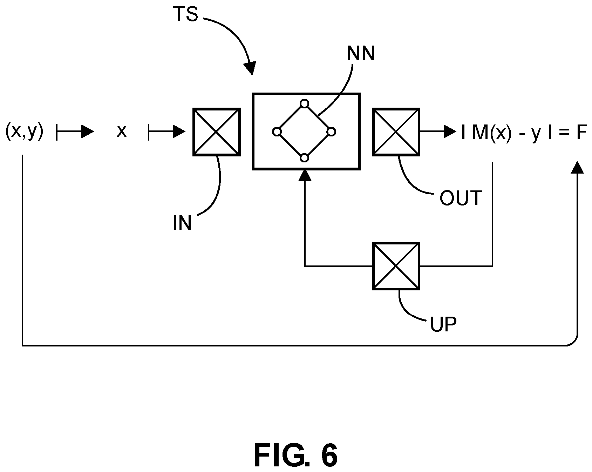

Fig. 6 shows components of a training systems TS that may be used during the training cycles.

The training system TS may be sued for adapting parameters, i.e. the weights of machine learning model M, such as in a convolutional neural network as discussed in Fig. 5, or other neural network-type model, or indeed non-neural network type ML models.

The training data admitted for training may comprises $k$ pairs of data $(x_k, y_k)$. k may run into the 10s, 100s or 1000s or larger still such as in order of $10^5$. The new training data comprises for each pair $k$ (the index $k$ is not related to the index used above to designate generation of feature maps), training input data $x_k$ and an associated target $y_k$. The training data is thus organized in pairs $k$ in particular for supervised learning schemes as mainly envisaged herein. However, it should be noted that non-supervised learning schemes are not excluded herein.

In the training phase, weights $\theta$ of the model NN represent a parameterization $M^\theta$, and it is the object of the training system TS to optimize and hence adapt the parameters $\theta$ based on the training data $(x_k, y_k)$ pairs. In other words, the learning can be formulized mathematically as an optimization scheme where a cost/loss function $F$ is minimized although the dual formulation of maximizing a utility function may be used instead.

Assuming for now the paradigm of a cost function F, this measures the aggregated residue(s), that is, the error incurred between data estimated by the neural network model NN and the targets as per some or all of the training data pairs $k$ in a batch for example:

$$argmin_{\theta} F = \sum_k \| M^\theta(x_k), y_k \| \qquad (3)$$

In eq. (3), function $M_\theta()$ denotes training output, the result of the model NN applied to input x. A suitable similarity measure $\|-\|$ is used to measure the difference, also referred to herein as residue, between the actual training output $M^\theta(x_k)$ produced by the model M, and the desired target $y_k$. When the model M is a classifier as mainly envisaged herein, each summand in (3) may be formulated in terms of cross-entropy or Kullback-Leibler divergence. In a multi-class classifier, each such summand may be formulated as a sum of cross-entropies over the envisaged classes.

In training of an NN model M, the training input data $x_k$ of a training pair is propagated through the initialized or pre-trained network M. Specifically, the training input $x_k$ for a $k$-th pair is received at an input IL, passed through the model and is then output at output OL as output training data $M^\theta(x)$.

The output training data $M(x_k)$ is an estimate for target $y_k$ associated with the applied input training image data $x_k$. In general, there is an error between this output $M(x_k)$ and the associated target $y_k$ of the presently considered $k$-th pair. An optimization scheme such as backward/forward propagation or other gradient based methods may then be used to adapt the parameters $\theta$ of the model M so as to decrease the aggregated residue the considered pair $(x_k, y_k)$ or a subset (batch) of training pairs. including in particular at least in parts the newly admitted (filtered) training data.

After one or more iterations in a first, inner, loop in which the parameters $\theta$ of the model are updated by updater UP for the current pair or pairs $(x_k, y_k)$, the training system TS enters a second, an outer, loop where a next training data pair $x^{k+1}, y^{k+1}$ or the next batch of training data is processed accordingly. The structure of updater UP depends on the optimization scheme used. For example, the inner loop as administered by updater UP may be implemented by one or more backward passes in a backpropagation algorithm. While adapting the parameters, the aggregated, for example summed, residues of all the training pairs making up the current batch, to improve the objective function. The aggregated residue can be formed by configuring the objective function $F$ as in eq. (3).

As mentioned, processing during training may be one by one processing for each pair, or processing is preferably per batch.

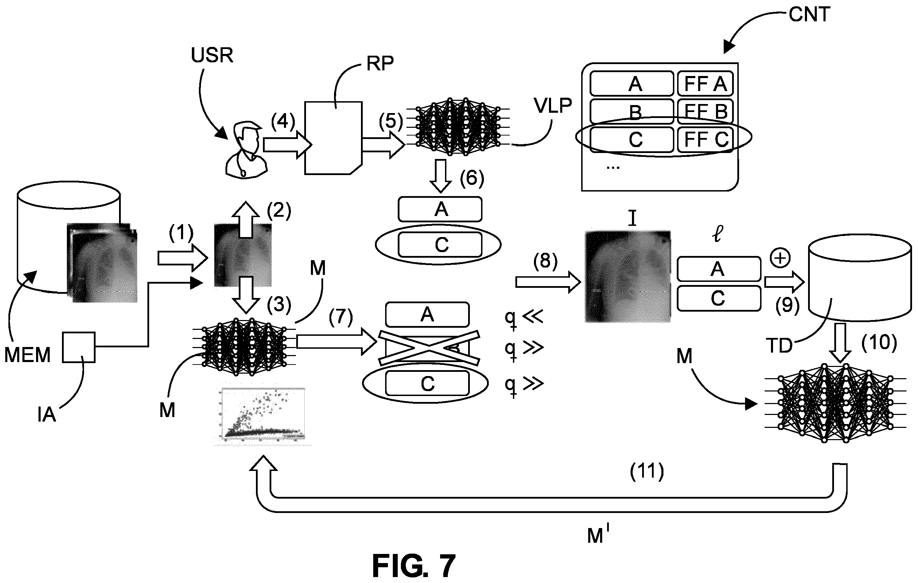

The training system as shown in Fig. 7 can be considered for all learning schemes, in particular supervised schemes. Unsupervised learning schemes may also be envisaged herein in alternative embodiments. GPUs may be used to implement the training system TS. The fully trained machine learning model M for the current cycle may be stored in one or more memories MEM. The trained model may be made available in a cloud service.

Fig. 7 shows is a schematic illustrative overview of how the proposed system SYS may be embedded into a clinical data flow. The deployment of the in-product learning system SYS as proposed herein may occur in a clinical environment. Images are supplied (1) by imager IA or retrieved from storage MEM. The image(s) may be interpreted (2), (4) by a radiologists USR (with or without ML-assistance), resulting in radiology report(s) RP. The report(s) RP may be subsequently analyzed (5) by an NLP model to extract (6) labels A, C. The labels may be correlated with the findings of the ML model M of system SYS. The system SYS determines if a particular image and its label should be included in the next training cycle. Fig. 7 depicts an example where the radiologists USR reports (6) abnormality A and C. The model M of system SYS finds (7) class A with high uncertainty, but potentially still useful, whilst classes B and C, are also of high uncertainty, but potentially harmful. Since label B was an outlier (and not reported by the radiologist USR), it is not admitted into the training data set. Both, label A and label C, are assigned (8) to the image, and the so labeled image is included (9) in the training data set, as label A with high uncertainty, and label C is marked as a rare disease/abnormality. The global class counter CNT operates to adapt the threshold T. The counts are used to keep track what is considered rare, and hence the uncertainty requirement T may be relaxed. In the example of Fig. 7, the inclusion decision for C is also strengthened by the reported label of the radiologist. However, the system SYS could also use flagging of a rare finding that was previously unreported by the radiologist for a manual review. When the newly inserted data samples reach a certain threshold, the model is retrained (10) and subsequently redeployed (11) in the production environment.

The example X-ray imagery in Figs. 4,7 is provided by the NIH Clinical Center, Bethesda, Maryland, and is available at *https://nihcc.app.box.com/v/ChestXray-NIHCC.* See also the paper by X. Wang, Y. Peng, L. Lu, Z. Lu, M. Bagheri and R. M. Summers, "ChestX-Ray8: Hospital-Scale Chest X-Ray Database and Benchmarks on Weakly-Supervised Classification and Localization of Common Thorax Diseases", 2017 IEEE Conference on Computer Vision and Pattern Recognition (CVPR), 2017, pp. 3462-3471, doi: 10.1109/CVPR.2017.369.

Reference is now made to the flow charts in Figs. 8 and 9 to further illustrate methods of operation of the above-described system. It will be understood however that the method steps described below are not necessarily tied to the architecture discussed above and may be understood as a teaching in their own right.

Referring first to the flow chart in Fig. 8, this illustrates steps of a computer-implemented method configured to facilitate a dynamic learning set-up for a machine learning model. The method implements filtering of imagery processed by the (trained) machine learning model during deployment to compile new training data for retraining of the model in a subsequent learning cycle. The imagery is filtered to improve performance of the model.

Referring now to steps of the method in more detail, at step S810 an input image is received during deployment.

At step S820 the machine learning model, trained in a previous training cycle based on training data, is used to classify the received input image into one or more, of one or more classes. The output of the model is a classification score vector as described above.

At step S830, in addition to the classification score, an uncertainty value is computed for the respective classification result per class. The uncertainty value may be computed by the model. or may be computed by an external entity. The computation is based on the classification score and parameters of the model.

At step S840, which is optional, a counter is set to count and track the number of cases classified per class. Optionally, only cases with a minimum classification score p are considered.

At step S850 the uncertainty value computed for the respective classification result per class is analyzed to determine, for the input image and per class, whether the input image is useful as a future training image of that class. The analysis may include comparing the current uncertainty value against uncertainty values for previously classified imagery S' processed by the machine learning model that were classified into the given class. A thresholding or other criterion may be used to implemented by a measure $\mathbb{Q}$. The measure $\mathbb{Q}$ may measure the magnitude of deviation of the current uncertainty value from the previous uncertainty values in S' as presented by the threshold or other criteria. Alternatively, it is merely a binary indication that is of interest, and the measure Q merely measures whether there is deviation or not. A deviation may be seen as indicative of the image being harmful for training. If there is no deviation, the input image may be deemed useful.

Specifically, based on the measure response as obtained in the analysis of step S850, a decision may be made at step 850a to admit the current image into a future training data cycle for retraining the model. At step S850b, if the uncertainty does not meet the criteria as formulate by measure Q, the current image is rejected and not allowed to be used in the next (or any other) training cycle.

The rejected, not admitted image, may be displayed, or a message may be generated and displayed, to bring the rejection to the user's attention for example. The user/clinician may then choose to review the rejected image. The clinician may approve the rejection or may override this and still admit the image for a retraining cycle. At step S860, the input image flagged for admittance into a next training cycle may be added to the current training data set. One or more training data batches may be formed to include a mix of previous and newly admitted training images for example.

At step S870 the counter for a given class is checked and the criterion used in step S840 for the analysis may be adjusted. The criterion for allowing images to be admitted for training may be relaxed. The criterion may be formulated in terms of thresholding, and the thresholding may be adjusted in dependence of the current class count. For low counts, the threshold may be increased and decreased with growing counts. In particular, for classes with a low occurrence/count rate the thresholding is relaxed.

This over time adaptation/relaxation of the criteria allows refined processing. Future imagery that would otherwise be found not useful/harmful for a given class, is now still admitted thanks to the relaxation, as such imagery may be representative of rare disease or category.

At step S880, the number of training data specimens admitted is checked. If this number is sufficiently high, given a threshold, and/or the admitted imagery is of sufficient demographic variation, re-training of the model is triggered, or a message/signal is generated to indicate to user that re-training could be triggered.

It will be understood that the above steps S820-S870 may be repeated for some or each class, and/or for each newly received image. Some images may be useful for same class(es), but harmful for other(s). The admitted training images may be re-labelled. so that only the (one or more) label remains for which the image has been found useful. Labels for which harmfulness has been found, are removed. More than one label may be awarded for admitted image. If the given image does not satisfy the criteria for any class, the image is flagged as not useful/as harmful for training, and will not be admitted in to any future training cycle.

The above-mentioned order of steps S840-S870 may be varied, but step S840 preferably occurs before step S870.

Furthermore, it will be understood herein that the above-described steps may depend on the uncertainties and the criteria are coded. For example, according to one coding scheme, a relaxation of the thresholding may entail lowering of a respective threshold used in the analysis step S840. This, the manner in which the thresholding or applicable criterion is adjusted in order to effect the relaxation, may depend on the particular encoding used. In particular, opposed encodings are also envisaged, in which case a relaxation may entail increasing the respective threshold. In general, the relaxation as used herein is configured so as to increase the likelihood that an image is admitted into the future training cycle.

As a further refinement of the Q measure used above for distinguishing between useful/harmful imagery, respective scores (referred to herein as alpha and beta scores) may be computed herein. The alpha scores measures/quantifies to what extent the given input image is useful for training, and the best score measures/quantifies to what extent the given input image is harmful for training. Thus, the beta scores may be computed in one embodiment by re-entering into training phase in respect of the image for which a beta score is required. A user interface may be provided that allows the user to specifically request such a re-entering.

In embodiments, the uncertainty $q$ may be decomposed into two parts, the alpha and beta scores by using an additional step in the model training process. More precisely, an additional output is added to the neural network or other ML model type that learns explicitly the "beta scores". The loss function aims to "explain away" the uncertainty $q$ associated with an input image. In the end of this additional process, uncertainty q is decomposed in two parts based on the alpha and beta values. Thus, a new loss function may be formulated as:

$$\hat{F} = F + \beta \tag{4}$$

Thus, the new loss function $\hat{F}$ is the sum of the original loss function $F$ (see above at eq (3)) and a "beta term", since the original uncertainty value $q$, associated the input image, implies that the network cannot converge to a "steady output" for this image. Inclusion of the beta term ($\beta$) may entail additional iterations in the training phase, such as additional gradient descent steps. On termination of the iterations (once sufficient convergence is detected), the value of the beta term $\beta$ may be supplied as an additional output to obtain an estimate for the beta score. This may help the network to identify the separation of the uncertainty $q$ into alpha and beta. After this step the uncertainty $q$ becomes essentially a weighted linear combination of alpha and beta scores.

Thus, the beta scores are explicitly learned by the machine learning model, such as a deep learning model (deep l. This may be beneficial since (deep) ML models are known to have superior performance for image analysis compared to more traditional machine learning or statistical techniques. In this manner, this approach allows for more accurate identification of the images that are more appropriate for improving the model performance. The above-mentioned adaptive thresholding (such as at eq (2) or other) is preferably applied to the beta score.

Steps involved in the re-training of the model are now explained with reference to the flow chart in Fig. 9.

The retraining steps are essentially the same as those for the first or earlier training cycle, but differ in the composition of the training data. In the first training cycle, the training data including suitably labeled imagery from historical exams. The imagery is obtained from medical image repositories and are labelled manually by a human domain expert, or the labeling is done automatically by NLP processing of medical reports or patient health records associated with the stored imagery.

If the classes are known a priori, a simple keyword searches may suffice, and complex NLP processing may not necessarily be required. In NLP processing, the classes may not be known beforehand. The NLP analyses natural language text to ascertain findings from the grammar structure and semantics of the texts in the reports to detect the classes.

In retraining, the training data is supplemented by the filtered training data set taken from the deployment experience data set that accumulated since the earlier (eg, first), training cycle.

After triggering retraining at step S880, at step S910 training data for the new training cycle is received.

The training data for the new cycle may include exclusively the filtered data as obtained by method of Fig. 8 (referred to herein as the "new training data"), or the training data may include a mix of newly filtered data and data from past cycle(s), referred to herein as the "old training data". Original training data from the first training cycle may be included.

At step S920, the new training data, an optionally older training data, is applied to an intimal ML model or to one pretrained in a previous training cycle. The training data may be applied one by one or in subsets at once, such as in batches. The batches may include a mix of new and old training data. Alternatively, the batch incudes only new data. Plural batches may be formed an these are applied in sequence or in parallel. Batchwise training is preferred herein.

At step S930 parameters $\theta$ of the ML model are adapted. The adaptation may be done iteratively in a training algorithm. The training algorithm may be configured to improve an objective function F, such as cost function.

At step S940 a stopping condition is evaluated. The stopping condition may include a set number of iterations or once the objective function fulfills a condition such as the cost drops below a predefined threshold. The stopping condition may include a predefined level of convergence of the iteration results.

For example, at step S920, one or more training input(s) $x_k$ is applied to an initialized machine learning model NN to produce a or respective training outputs.

A deviation, or residue, of the respective training output $M(x_k)$ from the associated target $y_k$ is quantified by the cost function $F$. One or more parameters of the model are adapted at step S930 in one or more iterations in an inner loop to improve the cost function. For instance, the model parameters are adapted to decrease an aggregation of the residues as measured by the cost function. The parameters may include in particular weights of convolutional operators, in case a convolutional NN model M is used. The parameters of the model are adapted so that the aggregated residues of all pairs considered are decreased, in particular minimized. The cost function quantifies the aggregated residues. Forwardbackward propagation or similar gradient-based techniques may be used in the inner loop.

The training method then returns in an outer loop to step S910 where the next pair of training data or a batch of training data is fed in.

More generally, the parameters of the model NN are adjusted to improve objective function $F$ which is either a cost function or a utility function. In embodiments, the cost function is configured to the measure the aggregated residues. In embodiments the aggregation of residues is implemented by summation over all or some residues for all pairs considered in the given batch. The method may be implemented on one or more general-purpose processing units TS, preferably having processors capable for parallel processing to speed up the training.

The components of the training system TS may be implemented as one or more software modules, run on one or more general-purpose processing units PU such as a workstation associated with the imager IA, or on a server computer associated with a group of imagers.

The components of the system SYS may be implemented as one or more software modules, run on one or more general-purpose processing units PU such as a workstation associated with the imager IA, or on a server computer associated with a group of imagers.

Alternatively, some or all components of the system SYS may be arranged in hardware such as a suitably programmed microcontroller or microprocessor, such an FPGA (field-programmable-gate-array) or as a hardwired IC chip, an application specific integrated circuitry (ASIC), integrated into the imaging system IA. In a further embodiment still, the system SYS may be implemented in both, partly in software and partly in hardware.

The different components of the system SYS may be implemented on a single data processing unit PU. Alternatively, some or more components are implemented on different processing units PU, possibly remotely arranged in a distributed architecture and connectable in a suitable communication network such as in a cloud setting or client-server setup, etc.

One or more features described herein can be configured or implemented as or with circuitry encoded within a computer-readable medium, and/or combinations thereof. Circuitry may include discrete and/or integrated circuitry, a system-on-a-chip (SOC), and combinations thereof, a machine, a computer system, a processor and memory, a computer program.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit, which might also be part of an embodiment of the present invention. This computing unit may be adapted to perform or induce a performing of the steps of the method described above. Moreover, it may be adapted to operate the components of the above-described apparatus. The computing unit can be adapted to operate automatically and/or to execute the orders of a user. A computer program may be loaded into a working memory of a data processor. The data processor may thus be equipped to carry out the method of the invention.

This exemplary embodiment of the invention covers both, a computer program that right from the beginning uses the invention and a computer program that by means of an up-date turns an existing program into a program that uses the invention.

Further on, the computer program element might be able to provide all necessary steps to fulfill the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section.

A computer program may be stored and/or distributed on a suitable medium (in particular, but not necessarily, a non-transitory medium), such as an optical storage medium or a solidstate medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

In the claims, the word "comprising" does not exclude other elements or steps, and the indefinite article "a" or "an" does not exclude a plurality. A single processor or other unit may fulfill the functions of several items re-cited in the claims. The mere fact that certain measures are re-cited in mutually different dependent claims does not indicate that a combination of these measures cannot be used to advantage.

## Claims

1. A computer-implemented system (SYS) for providing training data,

   wherein the system is configured to receive at least one classification result for a class from plural pre-defined classes ($C_j$),
   wherein the at least one classification result has been produced by a trained machine learning model (M) in response to processing an input image, and
   wherein the system (SYS) comprises a decision logic (DL), wherein the decision logic is configured to:

   analyze input data ($p_i$, $q_i$) comprising a received classification result score ($p_i$) and an uncertainty value ($q_i$) associated with the classification result score of the at least one classification result, and
   output, for the at least one received classification result, an associated indication whether the input image is or is not useful for re-training the trained machine learning model (M) in respect of the said class,
   wherein the usefulness is computed based on the uncertainty value ($q_i$) associated with the classification result score ($p_i$) of the input image and the uncertainty values of previously classified images,
   wherein the analysis by the system is based on a deployment experience set, and
   wherein the deployment experience set comprises all images processed by the trained machine learning model (M) since the last training of the trained machine learning model (M).

2. System of claim 1, wherein the input data ($p_i$, $q_i$) for the images that have been processed by the trained machine learning model (M) since the last training is added to the said deployment experience set.

3. System of claim 1 or 2, wherein the analysis by the system is based on at least one criterion and wherein the criterion is adaptable based on the size of the deployment experience set.

4. System of claim 3, including a counter (CNT) configured to track, per class, the said size of a subset of said deployment experience set.

5. System of any one of claims 3-4, wherein the said criterion is relaxed so as to increase the number of future input images indicatable as useful for retraining.

6. System of any one of the previous claims, wherein the analysis by the system includes the system (SYS) performing an outlier analysis in respect of the uncertainty value (qi) relative to uncertainty values of the previous input data for the said class.

7. System of any one of the previous claims, wherein the system is configured to identify from a plurality of not useful images with similar classification result score (pi) and uncertainty value (qi) a new class, not among the pre-defined classes ($C_j$).

8. System of claim 7, where the identifying is based on an n-dimensional outlier analysis of the uncertainty value (qi), where *n* is greater than or equal to 2 and where

*n* is a count of a number of classes;

*n* is a count of a number of images that were already assessed to be of high uncertainty;

*n* is a count of a number of region of images with high uncertainty scores; or

*n* relates to a respective proportion/frequency:

wherein optionally the uncertainty value (qi) is based on a latent space of the trained machine learning model (M) associated with the different classes of the trained machine learning model.

9. System of any one of the previous claims, wherein the decision logic (DL) is configured to trigger retraining by a training system TS of the trained machine learning model (M), based on a training data set including one or more such input images indicated as useful for retraining.

10. System of any one of previous claims, wherein the trained machine learning model (M) is a classifier of any one of the following types: artificial neural network model, support vector machine, decision tree, random forest, k-nearest neighbor, naive Bayes and linear discriminate analysis.

11. System of any one of the previous claims, wherein the input image is of any one of more of the following types: X-ray, magnetic resonance, ultrasound, nuclear.

12. The system of any one of the preceding claims further comprising:

- an imaging apparatus supplying the input image, and/or
- a memory from which the input image is receivable.

13. The system of claim 9, wherein the trained machine learning model (M) is used to process the input image and produce as output a medical result (RS).

14. A computer-implemented method for providing training data, the method comprising:

receiving (S810) at least one classification result for a class from plural pre-defined classes ($C_j$), the at least one classification result produced by a trained machine learning model (M) in response to processing an input image, analyzing (S850) input data ($p_i, q_i$) comprising a received classification result score ($p_i$) and an uncertainty value ($q_i$) associated with the classification result score of the at least one classification result, and outputting (S850a,b), for the at least one received classification result, an associated indication whether the input image is or is not useful for re-training the trained machine learning model in respect of the said class,

wherein the usefulness is computed based on the uncertainty value ($q_i$) associated with the classification result score ($p_i$) of the input image and the uncertainty values of previously classified images, wherein the analysis is based on a deployment experience set, and wherein the deployment experience set comprises all images processed by the trained machine learning model (M) since the last training of the trained machine learning model (M).

15. A computer program, which, when the program is executed by at least one processing unit (PU), causes the processing unit (PU) to perform the method of claim 14.

**Patentansprüche**

1. Computerimplementiertes System (SYS) zum Bereitstellen von Trainingsdaten,

wobei das System konfiguriert ist, um mindestens ein Klassifizierungsergebnis für eine Klasse aus mehreren vordefinierten Klassen ($C_j$) zu empfangen, wobei das mindestens eine Klassifizierungsergebnis von einem trainierten maschinellen Lernmodell (M) als Reaktion auf das Verarbeiten eines Eingangsbildes erzeugt worden ist, und wobei das System (SYS) eine Entscheidungslogik (DL) umfasst, wobei die Entscheidungslogik konfiguriert ist, um:

Eingangsdaten ($p_i, q_i$) zu analysieren, die eine empfangene Klassifizierungsergebnisbewertung ($p_i$) und

einen Unsicherheitswert ($q_i$) umfassen, welcher der Klassifizierungsergebnisbewertung des mindestens einen Klassifizierungsergebnisses zugeordnet ist, und

für das mindestens eine empfangene Klassifizierungsergebnis eine zugeordnete Angabe auszugeben, ob das Eingangsbild für ein erneutes Trainieren des trainierten maschinellen Lernmodells (M) in Bezug auf die Klasse nützlich ist oder nicht,

wobei die Nützlichkeit basierend auf dem Unsicherheitswert ($q_i$), welcher der Klassifizierungsergebnisbewertung ($p_i$) des Eingangsbildes zugeordnet ist, und den Unsicherheitswerten zuvor klassifizierter Bilder berechnet wird,

wobei die Analyse durch das System auf einem Einsatzerfahrungssatz basiert, und wobei der Einsatzerfahrungssatz alle Bilder umfasst, die seit dem letzten Training des trainierten maschinellen Lernmodells (M) von dem trainierten maschinellen Lernmodell (M) verarbeitet worden sind.

2. System nach Anspruch 1, wobei die Eingangsdaten ($p_i$,$q_i$) für die Bilder, die seit dem letzten Training von dem trainierten maschinellen Lernmodell (M) verarbeitet worden sind, zu dem Einsatzerfahrungssatz hinzugefügt werden.

3. System nach Anspruch 1 oder 2, wobei die Analyse durch das System auf mindestens einem Kriterium basiert, und wobei das Kriterium basierend auf der Größe des Einsatzerfahrungssatzes anpassbar ist.

4. System nach Anspruch 3, das einen Zähler (CNT) beinhaltet, der konfiguriert ist, um pro Klasse die Größe einer Teilmenge des Einsatzerfahrungssatzes zu verfolgen.

5. System nach einem der Ansprüche 3-4, wobei das Kriterium gelockert wird, um die Anzahl künftiger Eingangsbilder, die als für ein erneutes Training nützlich angebbar sind, zu erhöhen.

6. System nach einem der vorstehenden Ansprüche, wobei die Analyse durch das System beinhaltet, dass das System (SYS) eine Ausreißeranalyse in Bezug auf den Unsicherheitswert ($q_i$) im Verhältnis zu Unsicherheitswerten der vorherigen Eingangsdaten für die Klasse durchführt.

7. System nach einem der vorstehenden Ansprüche, wobei das System konfiguriert ist, um aus einer Vielzahl nicht nützlicher Bilder mit ähnlicher Klassifizierungsergebnisbewertung ($p_i$) und ähnlichem Unsicherheitswert ($q_i$) eine neue Klasse zu identifizieren, die sich nicht unter den vordefinierten Klassen ($C_j$) befindet.

8. System nach Anspruch 7, wobei das Identifizieren auf einer n-dimensionalen Ausreißeranalyse des Unsicherheitswertes ($q_i$) basiert, wobei *n* größer oder gleich 2 ist, und wobei

*n* eine Zählung einer Anzahl von Klassen ist;
*n* eine Zählung einer Anzahl von Bildern ist, bei denen bereits eine hohe Unsicherheit festgestellt worden ist;
*n* eine Zählung einer Anzahl von Bildbereichen mit hohen Unsicherheitsbewertungen ist; oder
*n* sich auf eine/n jeweilige/n Anteil/Häufigkeit bezieht;
wobei der Unsicherheitswert ($q_i$) optional auf einem latenten Raum des trainierten maschinellen Lernmodells (M) basiert, der den unterschiedlichen Klassen des trainierten maschinellen Lernmodells zugeordnet ist.

9. System nach einem der vorstehenden Ansprüche, wobei die Entscheidungslogik (DL) konfiguriert ist, um ein erneutes Training durch ein Trainingssystem TS des trainierten maschinellen Lernmodells (M) basierend auf einem Trainingsdatensatz auszulösen, der ein oder mehrere solcher Eingangsbilder beinhaltet, die als für das erneute Training nützlich angegeben werden.

10. System nach einem der vorstehenden Ansprüche, wobei das trainierte maschinelle Lernmodell (M) ein Klassifikator eines der folgenden Typen ist: künstliches neuronales Netzwerkmodell, Unterstützungsvektormaschine, Entscheidungsbaum, Zufallswald, k-Nächste-Nachbarn, Naive Bayes- und lineare Diskriminanzanalyse.

11. System nach einem der vorstehenden Ansprüche, wobei das Eingangsbild eines oder mehrere der folgenden Typen ist: Röntgen, Magnetresonanz, Ultraschall, Nuklear.

12. System nach einem der vorstehenden Ansprüche, weiter umfassend:

- eine Bildgebungseinrichtung, die das Eingangsbild liefert, und/oder
- einen Speicher, aus dem das Eingangsbild empfangbar ist.

13. System nach Anspruch 9, wobei das trainierte maschinelle Lernmodell (M) verwendet wird, um das Eingangsbild zu verarbeiten und als Ausgabe ein medizinisches Ergebnis (RS) zu erzeugen.

14. Computerimplementiertes Verfahren zum Bereitstellen von Trainingsdaten, wobei das Verfahren umfasst:

Empfangen (S810) mindestens eines Klassifizierungsergebnisses für eine Klasse aus mehreren vordefinierten Klassen ($C_j$), wobei das mindestens eine Klassifizierungsergebnis von einem trainierten maschinellen Lernmodell (M) als Reaktion auf Verarbeiten eines Eingangsbildes erzeugt worden ist,

Analysieren (S850) von Eingangsdaten ($p_i,q_i$), die eine empfangene Klassifizierungsergebnisbewertung ($p_i$) und einen Unsicherheitswert ($q_i$) umfassen, welcher der Klassifizierungsergebnisbewertung des mindestens einen Klassifizierungsergebnisses zugeordnet ist, und

Ausgeben (S850a,b), für das mindestens eine empfangene Klassifizierungsergebnis, einer zugeordneten Angabe, ob das Eingangsbild für ein erneutes Trainieren des trainierten maschinellen Lernmodells in Bezug auf die Klasse nützlich ist oder nicht,

wobei die Nützlichkeit basierend auf dem Unsicherheitswert ($q_i$), welcher der Klassifizierungsergebnisbewertung ($p_i$) des Eingangsbildes zugeordnet ist, und den Unsicherheitswerten zuvor klassifizierter Bilder berechnet wird,

wobei die Analyse auf einem Einsatzerfahrungssatz basiert, und

wobei der Einsatzerfahrungssatz alle Bilder umfasst, die seit dem letzten Training des trainierten maschinellen Lernmodells (M) von dem trainierten maschinellen Lernmodell (M) verarbeitet worden sind.

15. Computerprogramm, das, wenn das Programm von mindestens einer Verarbeitungseinheit (PU) ausgeführt wird, die Verarbeitungseinheit (PU) veranlasst, das Verfahren nach Anspruch 14 durchzuführen.

**Revendications**

1. Système mis en œuvre par ordinateur (SYS) pour la fourniture de données de formation,

dans lequel le système est configuré pour recevoir au moins un résultat de classification pour une classe parmi plusieurs classes prédéfinies ($C_j$),

dans lequel le au moins un résultat de classification a été produit par un modèle d'apprentissage automatique formé (M) en réponse au traitement d'une image d'entrée, et

dans lequel le système (SYS) comprend une logique de décision (DL), dans lequel la logique de décision est configurée pour :

analyser des données d'entrée ($p_i$, $q_i$) comprenant un score de résultat de classification reçu ($p_i$) et une valeur d'incertitude ($q_i$) associée au score de résultat de classification du au moins un résultat de classification, et

délivrer en sortie, pour au moins un résultat de classification reçu, une indication associée indiquant si l'image d'entrée est ou non utile pour former à nouveau le modèle d'apprentissage automatique formé (M) par rapport à ladite classe,

dans lequel l'utilité est calculée sur la base de la valeur d'incertitude ($q_i$) associée au score de résultat de classification ($p_i$) de l'image d'entrée et des valeurs d'incertitude d'images précédemment classées,

dans lequel l'analyse par le système est basée sur un ensemble d'expériences de déploiement, et dans lequel l'ensemble d'expériences de déploiement comprend toutes les images traitées par le modèle d'apprentissage automatique formé (M) depuis la dernière formation du modèle d'apprentissage automatique formé (M).

2. Système selon la revendication 1, dans lequel les données d'entrée ($p_i,q_i$) pour les images qui ont été traitées par le modèle d'apprentissage automatique formé (M) depuis la dernière formation sont ajoutées audit ensemble d'expériences de déploiement.

3. Système selon la revendication 1 ou 2, dans lequel l'analyse par le système est basée sur au moins un critère et dans lequel le critère est adaptable en fonction de la taille de l'ensemble d'expériences de déploiement.

4. Système selon la revendication 3, incluant un compteur (CNT) configuré pour suivre, par classe, ladite taille d'un sous-ensemble dudit ensemble d'expériences de déploiement.

5. Système selon l'une quelconque des revendications 3-4, dans lequel ledit critère est assoupli de manière à augmenter le nombre d'images d'entrée futures pouvant être indiquées comme utiles pour la nouvelle formation.

6. Système selon l'une quelconque des revendications précédentes, dans lequel l'analyse par le système inclut le système (SYS) réalisant une analyse de valeurs aberrantes par rapport à la valeur d'incertitude ($q_i$) en lien avec les aux valeurs d'incertitude des données d'entrée précédentes pour ladite classe.

7. Système selon l'une quelconque des revendications précédentes, dans lequel le système est configuré pour identifier, à partir d'une pluralité d'images non utiles avec un score de résultat de classification similaire ($p_i$) et une valeur d'incertitude ($q_i$), une nouvelle classe, qui n'est pas parmi les classes prédéfinies ($C_j$).

8. Système selon la revendication 7, dans lequel l'identification est basée sur une analyse de valeurs aberrantes à n dimensions de la valeur d'incertitude ($q_i$), *n* étant supérieur ou égal à 2 et dans lequel

   *n* est un compte d'un nombre de classes ;
   *n* est un compte d'un nombre d'images qui ont déjà été évaluées comme présentant une incertitude élevée ;
   *n* est un compte d'un nombre de régions d'images avec des scores d'incertitude élevés ; ou
   *n* se rapporte à une proportion/fréquence respective ;
   dans lequel, éventuellement, la valeur d'incertitude ($q_i$) est basée sur un espace latent du modèle d'apprentissage automatique formé (M) associé aux différentes classes du modèle d'apprentissage automatique formé.

9. Système selon l'une quelconque des revendications précédentes, dans lequel la logique de décision (DL) est configurée pour déclencher une nouvelle formation par un système de formation TS du modèle d'apprentissage automatique formé (M), sur la base d'un ensemble de données de formation incluant une ou plusieurs de ces images d'entrée indiquées comme utiles pour la nouvelle formation.

10. Système selon l'une quelconque des revendications précédentes, dans lequel le modèle d'apprentissage automatique formé (M) est un classificateur de l'un quelconque des types suivants : modèle de réseau neuronal artificiel, machine à vecteurs de support, arbre décisionnel, forêt aléatoire, k plus proches voisins, bayésien naïf et analyse de discrimination linéaire.

11. Système selon l'une quelconque des revendications précédentes, dans lequel l'image d'entrée est de l'un quelconque des types suivants : rayons X, résonance magnétique, ultrasons, nucléaire.

12. Système selon l'une quelconque des revendications précédentes, comprenant en outre :

   - un appareil d'imagerie fournissant l'image d'entrée, et/ou
   - une mémoire à partir de laquelle l'image d'entrée peut être reçue.

13. Système selon la revendication 9, dans lequel le modèle d'apprentissage automatique formé (M) est utilisé pour traiter l'image d'entrée et produire en sortie un résultat médical (RS).

14. Procédé mis en œuvre par ordinateur pour la fourniture de données de formation, le procédé comprenant :

   la réception (S810) d'au moins un résultat de classification pour une classe parmi plusieurs classes prédéfinies ($C_j$), le au moins un résultat de classification étant produit par un modèle d'apprentissage automatique formé (M) en réponse au traitement d'une image d'entrée,
   l'analyse (S850) de données d'entrée ($p_i$,$q_i$) comprenant un score de résultat de classification reçu ($p_i$) et une valeur d'incertitude ($q_i$) associée au score de résultat de classification du au moins un résultat de classification, et
   la production en sortie (S850a,b), pour le au moins un résultat de classification reçu, d'une indication associée indiquant si l'image d'entrée est ou non utile pour former à nouveau le modèle d'apprentissage automatique formé par rapport à ladite classe,
   dans lequel l'utilité est calculée sur la base de la valeur d'incertitude ($q_i$) associée au score de résultat de classification ($p_i$) de l'image d'entrée et des valeurs d'incertitude d'images précédemment classées,
   dans lequel l'analyse est basée sur un ensemble d'expériences de déploiement, et
   dans lequel l'ensemble d'expériences de déploiement comprend toutes les images traitées par le modèle d'apprentissage automatique formé (M) depuis la dernière formation du modèle d'apprentissage automatique formé (M).

15. Programme informatique qui, lorsque le programme est exécuté par au moins une unité de traitement (PU), amène l'unité de traitement (PU) à réaliser le procédé selon la revendication 14.

**FIG. 1**

**FIG. 2**

FIG. 3

**FIG. 4**

**FIG. 5**

**FIG. 6**

FIG. 7

**FIG. 8**

**FIG. 9**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **GHESU, F. C.** ; **GEORGESCU, B.** ; **MANSOOR, A.** ; **YOO, Y.** ; **GIBSON, E.** ; **VISHWANATH, R. S.** ; **COMANICIU, D.** Quantifying and leveraging predictive uncertainty for medical image assessment. *Medical Image Analysis*, 2021, vol. 68, 101855
- **AN, S.** ; **KIM, J.** ; **KANG, M.** ; **RAZAEI, S.** ; **LIU, X.** OAAE: Adversarial Autoencoders for Novelty Detection in Multi-modal Normality Case via Orthogonalized Latent Space. *arXiv:2101.02358*, 2021
- *Proposed Regulatory Framework for Modifications to Artificial Intelligence/Machine Learning (AI/ML)-Based Software as a Medical Device (SaMD) - Discussion Paper and Request for Feedback*, April 2019, https://www.fda.gov/media/122535/download
- **T. M. MITCHELL**. Machine Learning. McGraw-Hill, 1997, 6-1.2.1
- **M. HUBERT et al.** Outlier detection for skewed data. *Journal of Chemometrics*, March 2018, vol. 22 (3), 235-246
- **Y GAL et al.** Dropout as a Bayesian Approximation: Representing Model Uncertainty in Deep Learning. *arXiv:1506.02142 [stat.ML*, 04 October 2016
- **A KENDALL et al.** What Uncertainties Do We Need in Bayesian Deep Learning for Computer Vision?. *arXiv:1703.04977 [cs.CV*, 05 October 2017
- **M H SHAKER et al.** Aleatoric and Epistemic Uncertainty with Random Forests. *arXiv:2001.00893*, 03 January 2020
- **X. WANG** ; **Y. PENG** ; **L. LU** ; **Z. LU** ; **M. BAGHERI** ; **R. M. SUMMERS**. ChestX-Ray8: Hospital-Scale Chest X-Ray Database and Benchmarks on Weakly-Supervised Classification and Localization of Common Thorax Diseases. *2017 IEEE Conference on Computer Vision and Pattern Recognition (CVPR)*, 2017, 3462-3471